# EUROPEAN PATENT SPECIFICATION

(11) **EP 3 833 765 B1**
(45) Date of publication and mention of the grant of the patent: **14.01.2026**
(21) Application number: 18769250.4
(22) Date of filing: 09.08.2018
(51) Int. Cl.: C12N 15/86, A61K 35/76

(54) **ONCOLYTIC VIRUS CARRYING E-CADHERIN AND USES THEREOF**
ONKOLYTISCHES VIRUS MIT E-CADHERIN UND DESSEN VERWENDUNGEN
VIRUS ONCOLYTIQUE PORTANT UNE E-CADHÉRINE ET SES UTILISATIONS

(30) Priority: 09.08.2017 US 201762543328 P
(43) Date of publication of application: 16.06.2021
(73) Proprietor: Ohio State Innovation Foundation, Columbus, OH 43201 (US)
(72) Inventor: YU, Jianhua, Columbus OH 43201 (US); CALIGIURI, Michael, Columbus OH 43201 (US); XU, Bo, Columbus OH 43201 (US)
(74) Representative: Creek, Isobel Clare
(86) International application number: PCT/US2018/046064
(87) International publication number: WO 2019/032866

(56) References cited:
- J. HAN ET AL: "TGF Treatment Enhances Glioblastoma Virotherapy by Inhibiting the Innate Immune Response", CANCER RESEARCH, vol. 75, no. 24, 2 December 2015 (2015-12-02), US, pages 5273 - 5282, XP055513792, ISSN: 0008-5472, DOI: 10.1158/0008-5472.CAN-15-0894
- XUN XU ET AL: "The genomic sequence of the Chinese hamster ovary (CHO)-K1 cell line", NATURE BIOTECHNOLOGY, vol. 29, no. 8, 31 July 2011 (2011-07-31), pages 735 - 741, XP055223996, ISSN: 1087-0156, DOI: 10.1038/nbt.1932
- F. STRUYF ET AL: "Mutations in the N-Terminal Domains of Nectin-1 and Nectin-2 Reveal Differences in Requirements for Entry of Various Alphaherpesviruses and for Nectin-Nectin Interactions", JOURNAL OF VIROLOGY., vol. 76, no. 24, 15 December 2002 (2002-12-15), US, pages 12940 - 12950, XP055513808, ISSN: 0022-538X, DOI: 10.1128/JVI.76.24.12940-12950.2002
- JIHAN AKHTAR ET AL: "HVEM and Nectin-1 Are the Major Mediators of Herpes Simplex Virus 1 (HSV-1) Entry into Human Conjunctival Epithelium", INVESTIGATIVE OPTHALMOLOGY & VISUAL SCIENCE, vol. 49, no. 9, 1 September 2008 (2008-09-01), US, pages 4026, XP055513701, ISSN: 1552-5783, DOI: 10.1167/iovs.08-1807
- DREES FRAUKE ET AL: "[alpha]-Catenin Is a Molecular Switch that Binds E-Cadherin-[beta]-Catenin and Regulates Actin-Filament Assembly", CELL, vol. 123, no. 5, 2 December 2005 (2005-12-02), pages 903 - 915, XP029159230, ISSN: 0092-8674, DOI: 10.1016/J.CELL.2005.09.021

## Description

### FIELD OF THE INVENTION

The disclosure provides oncolytic virus, in particular a herpes virus, comprising a heterologous gene encoding E-cadherin. The disclosure further provides pharmaceutical compositions that comprise such oncolytic herpes virus, and methods of treatment using such oncolytic herpes virus.

The references to methods of treatment of the human or animal body by surgery or therapy are to be interpreted as references to the compounds, pharmaceutical compositions and medicaments of the present invention for use in those methods.

### BACKGROUND

Glioblastoma (GBM) is the most common primary brain malignancy. It is characterized by rapid growth, high invasion, genetic heterogeneity, chemotherapy resistance, and a high frequency of recurrence (Wen et al., 2008, NEJM359, 492-507). GBM affects the brain, the most complex and delicate organ of the human body, further limiting treatment options. Despite recent advances in understanding the molecular mechanisms of GBM and the development of new therapeutics, the median overall survival of GBM patients is still only 14.6 months (Stupp et al., 2009, European Organisation for, T. Treatment of Cancer Brain, G. Radiation Oncology, G. National Cancer Institute of Canada Clinical Trials, The Lancet. Oncology 10, 459-466). More importantly, this figure has not significantly improved in the past two decades.

Oncolytic viruses (OVs) comprise a promising biologic reagent currently in clinical investigation in humans for the treatment of many cancers, including GBM. Oncolytic herpes simplex virus-I (oHSV), the first genetically engineered OV, has been shown to be effective for the treatment of various cancers (Martuza et al., 1991, Science 252, 854-856; Pol et al., 1998, Oncoimmunology 5, el 115641 (2016); Toda, et al., Human gene therapy 9, 2177-2185; Kooby et al., 1999, FASEB journal: official publication of the Federation of American Societies for Experimental Biology 13, 1325-1334; Toyoizumi et al., 1999, Human gene therapy 10, 3013-3029). In October 2015, the FDA approved the first oHSV, Imlygic (Talimogene laherparepvec), permitting clinical use of oHSV in cancer treatment (Pol et al., 2016, Oncoimmunology 5, el 115641).

However, the current efficacy of oHSV in treating GBM is limited. The host innate immune response to oHSV infection impairs efficient viral replication and propagation within tumor tissues following oHSV administration, resulting in compromised therapeutic efficacy of oHSV against GBM (*see,* Kurozumi et al., 2007, Journal of the National Cancer Institute 99, 1768-1781; Ikeda et al., 2012, Nature Medicine 5, 881-887; Alvarez-Breckenridge et al., 2012, Nature Medicine 18, 1827-1834; Han et al., 2015, Cancer Research 75, 5273-5282). Administration of oHSV in the brain induced rapid recruitment and activation of NK cells, which accounted for obvious viral clearance and limited anti-tumor efficacy of oHSV in animal models (Alvarez-Breckenridge et al., 2012, Nature Medicine 18, 1827-1834 (2012); Han et al., 2015, Cancer Research 75, 5273-5282). Another critical hurdle for oHSV is limited intratumoral viral spread. In the environment of tightly jointed solid tumors including GBM, many factors such as extracellular matrix, areas of fibrosis and necrosis, and surrounding normal cells in the tumor bed as well as limited viral yields can limit intratumoral viral spread of oHSV (Kolodkin-Gal et al., 2008, Journal of Virology 82, 999-1010). This limited viral spread reduces the incidence of secondary infection, impairs viral replication and reproduction, shortens the action time of oHSV therapy, and results in unsatisfactory treatment efficacy of oHSV.

Previous studies demonstrated that NK cell depletion or inhibition significantly improved the efficacy of oHSV on treating GBM (Ikeda et al., 1999, Nature Medicine 5, 881-887; Fulci et al., 2006, Proceedings of the National Academy of Sciences of the United States of America 103, 12873-12878; Alvarez-Breckenridge et al., 2012, Nature medicine 18, 1827-1834; Alvarez-Breckenridge et al., 2012, Journal of Virology 86, 4566-4577; Han et al., 2015,Cancer Research 75, 5273-5282). Transient natural killer (NK) cell depletion with antibodies of either asialo-GM1 or TMβ1 significantly increased survival of mice bearing orthotopic GBM xenografts treated with oHSV (Alvarez-Breckenridge et al., 2012, Nature Medicine 18, 1827-1834). Consistent with this outcome, pretreatment with tumor growth factor β (TGFβ) prior to oHSV administration for GBM transiently was demonstrated to inhibit NK cells and improve oHSV therapeutic responses and survival outcomes (Han et al., 2015, Cancer Research 75, 5273-5282). However, systematic depletion or inhibition of NK cells inevitably impairs the antitumor effect of NK cells. Modulation of activating and/or inhibitory signals of those NK cells that specifically interact with oHSV-infected cells could be a better approach to selectively inhibit their antiviral activity.

Killer cell lectin-like receptor G1 (KLRG1) is an NK cell inhibitory receptor. Binding of KLRG1 to its ligand E-cadherin, which is encoded by the gene CDH1, inhibits lysis of E-cadherin-expressing cells by NK cells (Ito et al., 2006, Journal of Experimental Medicine 203, 289-295). KLGRl is also an inhibitory receptor in T cells (Grundemann, et al. 2006, J Immunol 176, 1311-1315). Besides its known function as a KLRG1 ligand, E-cadherin is well known as a calcium dependent cell-cell adhesion molecule that cooperates with Nectin-1 in the formation of cell-cell adherens junctions (Tachiban et al., 2000, The Journal of Cell Biology 150, 1161-1176; Drees et al., 2005, Cell 123, 903-915). As a herpes virus entry receptor, Nectin-1 is crucial for HSV-1 infection. The binding ofNectin-1 and HSV glycoprotein D (gD) triggers viral entry ofHSV. Loss ofNectin-1 expression in cells results in resistance or low susceptibility to HSV-1 infection (Akhtaret et al., 2008, Investigative Ophthalmology & Visual Science 49, 4026-4035; Xu et al., 2011, Nature Biotechnology 29, 735-741). E-cadherin junctions can recruit Nectin-1 to the cell surface, which may help to facilitate HSV-1 viral spread (Troyanovsky et al., 2015 Journal of Cell Science 128, 140-149). However, most GBM cells do not natively express E-cadherin (Lewis-Tuffin et al., 2010, PloS one 5, el3665).

Thus, there is a need in the art to develop oncolytic viral therapies, particularly oncolytic HSV therapies, that have improved virus spread within a solid tumor like GBM and that avoid depletion of virally infected cells by immune cells such as NK cells and T cells.

### SUMMARY

This invention provides reagents and methods for oncolytic viral therapies, which according to the invention are oncolytic HSV therapies, that have improved virus spread within a solidtumor like GBM and that avoid depletion of virally infected cells by immune cells such as NK cells. In particular embodiments according to the invention, provided herein is oncolytic herpes simplex virus (HSV) comprising a heterologous gene encoding E-cadherin.

In other embodiments, provided herein is a method for treating a subject having a tumor, the method comprising: administering to a subject in need thereof a therapeutic amount an oncolytic herpes simplex virus (HSV), comprising a heterologous gene encoding E-cadherin.

In other embodiments, provided herein is a pharmaceutical composition comprising an oncolytic HSV comprising a heterologous gene encoding E-cadherin and a pharmaceutically acceptable carrier or diluent.

In some embodiments, the heterologous gene is operably linked to a HSV promoter sequence which is selectively active during HSV replication. In some embodiments, the HSV promoter is a HSV immediate early promoter.

In some embodiments, the HSV further comprises an ICP 6-inactivating mutation or deletion. In some embodiments, the HSV further comprises a y-34.5 inactivating mutation or deletion.

In some embodiments, the tumor is a solid tumor such as a glioblastoma, an ovarian tumor or a breast tumor.

In some embodiments, the subject is a mammal. In some embodiments, the mammal is a human.

### BRIEF DESCRIPTION OF THE DRAWINGS

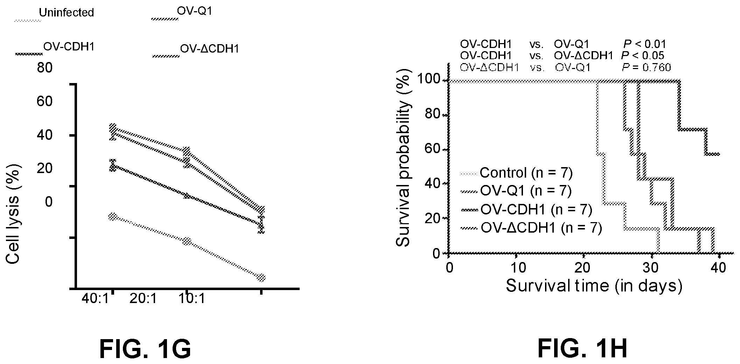

**Figures lA-lH** show oncolytic viral-E-cadherin (OV-CDHI) infection introduces E-cadherin expression to infected GBM cells. Figure l(A) illustrates a schematic map of oncolytic viruses used in the methods disclosed herein. Specifically, the top portion of the Figure illustrates a genetic map of wild type HSV-1; the middle portion illustrates a map of control oHSV, parental oHSV (OV-Ql), with deletion of y34.5, dysfunction ofICP6 and insertion of the green fluorescent protein (GFP) gene; and the bottom portion illustrates a map of OV-CDHI showing insertion of the human CDHI gene (encoding E-cadherin) driven by the viral IE4/5 promoter. The genetic maps of OV-11CDH1 shows the insertion of protein-coding sequence of mutant E-cadherin (IL-2Ra/E-cad), in which the original E-cadherin extracellular domain was replaced with the interleukin-2 receptor alpha chain (IL-2Ra) extracellular domain; the cytoplasmic domain remained intact. Figure l(B) illustrates immunoblotting performed with cell lysates from uninfected, OV-Ql- or OV-CDHI- infected GBM cells at 8 hours post infection (hpi). Figure l(C) illustrates immunoblotting performed with lysates from OV-CDHI- infected G1i36 or GBM30 cells at 0, 2, 4, 8, 12 hpi. Figure l(D) illustrates flow cytometry performed with uninfected, OV-Ql- or OV-CDHI-infected GBM cells at 8 hpi. Figure l(E) illustrates immunoblotting performed with cell lysates from OV-Ql, OV-CDHI and OV-11CDH1-infected U251 cells at 8 hpi using the antibody recognizing cytoplasmic domain of E-cadherin. Figure l(F) illustrates flow cytometry performed using OV-11CDH1 infected U251 cells at 8 hpi using anti-CD25. Figure l(G) illustrates NK cell cytotoxicity measured by ⁵¹Cr release from uninfected, OV-Ql-, OV-CDHI- and OV-11CDH1-infected Gli36 cells co-cultured with freshly isolated human primary NK effector cells at effector:target ratios of 40:1, 20:1, and 10:1 (***P* < 0.01, ****P* < 0.001, performed with three biological replicates). Figure 1(H) illustrates survival curves of Gl261N4-bearing mice treated with OV-Q1, OV-CDH1 or OV-ΔCDH1. The mice were intracranially injected with 1 × 10⁵ GL261N4 cells. 5 days after tumor implantation, mice were treated with OV-Q1, OV-CDH1 or OV-ΔCDH1 at the dose of 2 × 10⁵ PFU through intratumoral injection.

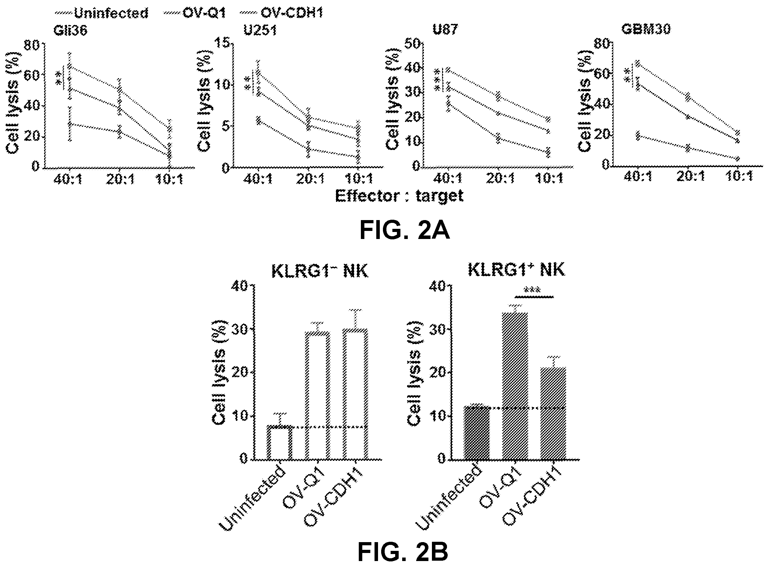

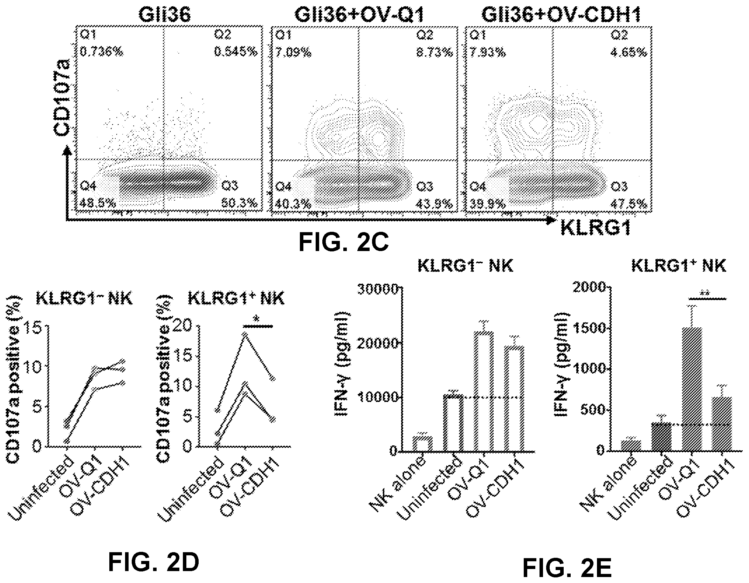

**Figures 2A-2E** **show OV-CDH1 infection reduces antiviral activity of human NK cells.** Figure 2(A) illustrates cytotoxicity of NK cells against different types of target cells measured by Chromium-51 (⁵¹Cr) release. Freshly isolated human bulk primary NK cells were used as effector cells. Uninfected, OV-Q1- or OV-CDH1-infected GBM cells were used as target cells. After labeling with ⁵¹Cr, target cells were cultured with effector cells at the effector:target ratios of 40:1, 20:1 and 10:1 for 4 hours. Figure 2(B) illustrates cytotoxicity of NK cells by measuring release of ⁵¹Cr. Human NK cells were sorted by flow cytometry to KLRG1⁺ and KLRG1⁻ subsets for effector cells. Gli36 cells were used as target cells. After ⁵¹Cr labeling, cells were co-cultured at the effector:target ratio of 30:1 for 4 hours. Figure 2(C) illustrates the results of flow cytometry assay of NK cell degranulation marker CD107a. Human NK cells were co-cultured with uninfected, OV-Q1- or OV-CDH1-infected Gli36 cells for 1 hour with anti-CD107a. Then secretion inhibitor was added to the co-culture for 4 hours. Cells were then washed and stained for flow cytometry assays. Figure 2(D) illustrates the statistical results of Figure 2(C) (*p<0.01, n=3). Figure 2(E) illustrates NK cell Interferon-γ (IFN-γ) secretion assays. Human bulk primary NK cells were co-cultured with the uninfected, OV-Q1- or OV-CDH1-infected Gli36 cells at ratios of 1:1 for 18 hours. IFN-γ secretion was measured by ELISA (**p<0.005, n=3).

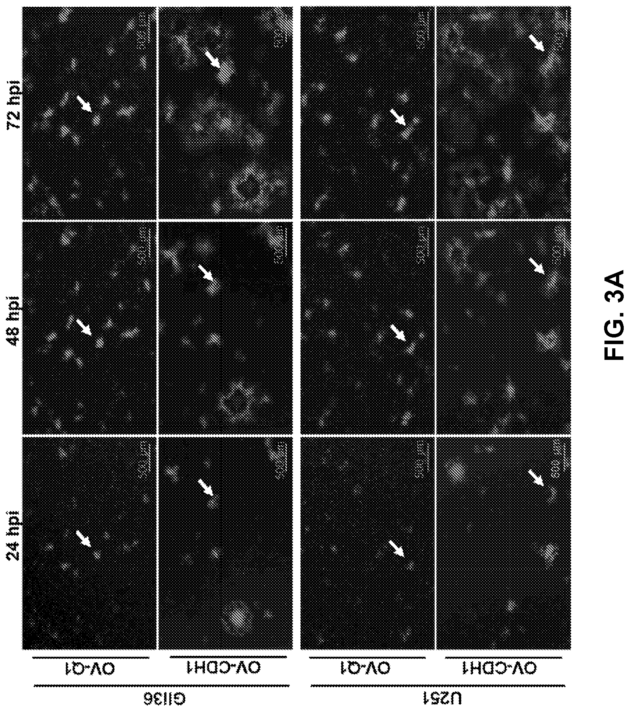

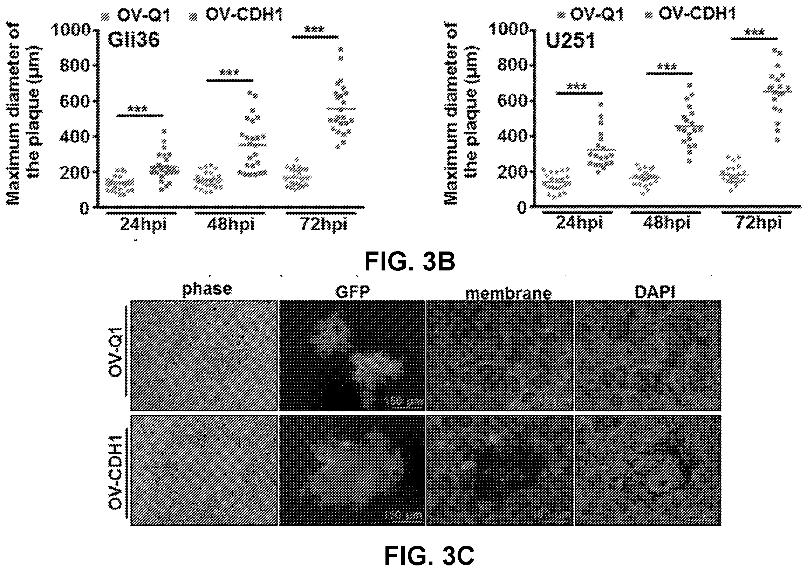

**Figures 3A-3C** **show cell-cell fusion facilitates viral spread of OV-CDH1.** Figure 3(A) illustrates monolayers of Gli36 and U251 cells infected with OV-Q1 or OV-CDH1 at the MOI of 0.005. At 48 hpi, the cells were imaged by microscopy at 24, 48 and 72 hpi. Each row shows the same vision of the cells at different times. The white arrows in each row point out the same plaque. Figure 3(B) shows measurements of plaque size. Three random views from each group were selected for measuring plaque size (p<0.001, n=3). Figure 3(C) shows monolayers of Gli36 cells infected with OV-Q1 or OV-CDH1 at an MOI of 0.005 and stained with membrane and nuclear dye at 48 hpi. EGFP: infected cells.

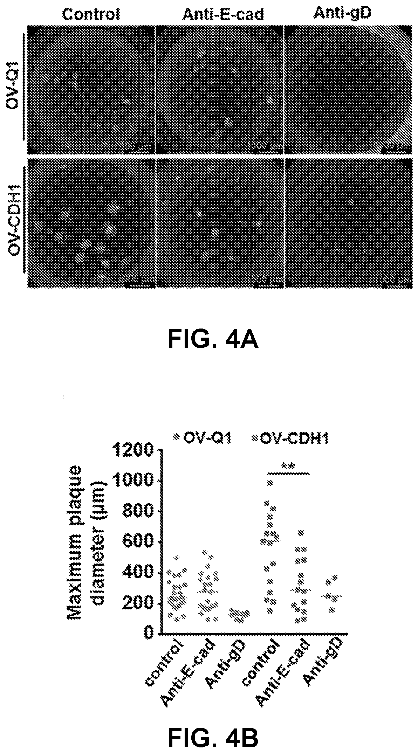

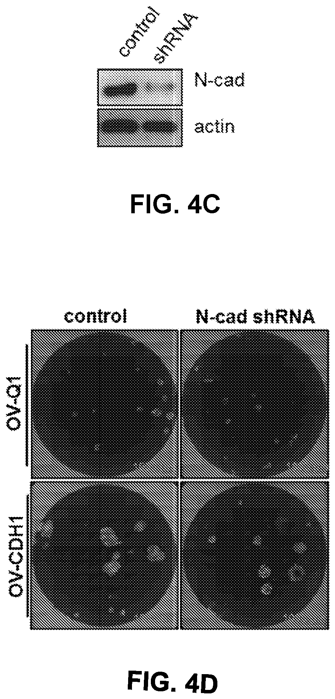

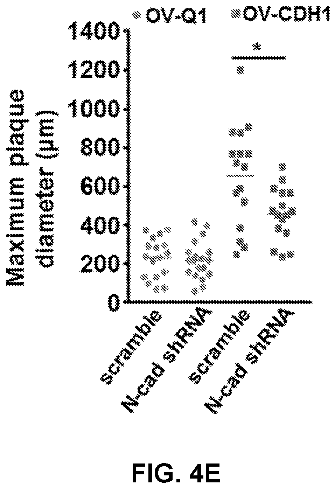

**Figures 4A-4E** **show cadherin interaction facilitates cell-to-cell infection of OV-CDH1.** Figure 4(A) illustrates monolayers of Gli36 cells infected with OV-Q1 or OV-CDH1 at the MOI of 0.005. At 2 hpi, media with E-cadherin blocking antibody or gD blocking antibody was replaced. Cells were imaged by microscopy at 48 hpi. Figure 4(B) illustrates plaque sizes, which for each group from Fig. 4(A) were measured from 3 randomly selected views (p<0.01, n=3). Figure 4(C) illustrates the expression of N-cadherin in Gli36 cells knocked down with shRNA (shCDH2). The protein expressions of N-cadherin were measured by immunoblotting. Figure 4(D) illustrates monolayers of Gli36 cells with or without N-cadherin knock-down were seeded in 96-well plates and infected with OV-Q1 or OV-CDH1 at the MOI of 0.005. Cells were imaged using a fluorescent microscope at 48 hpi. Scale bar, 1000 µm. Figure 4(E) illustrates quantification of plaque diameter (** *P* < 0.01, *** *P* < 0.001, sample sizes were indicated in the figures).

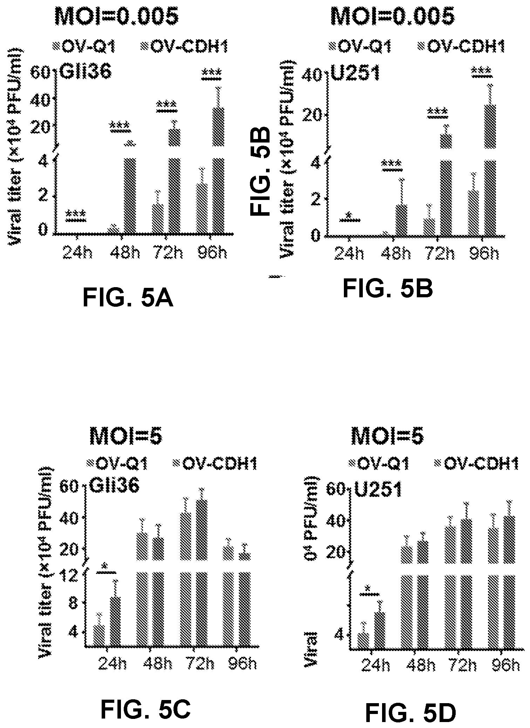

**Figures 5A-5D** **show increased viral production of OV-CDH1.** Figures 5(A) and Figure 5(B) illustrate Gli36 and U251 cells infected with OV-Q1 or OV-CDH1 at the MOI of 0.005. 24, 48, 72 and 96 hours after infection the supernatant viral titers from each group were measured by plaque assay (*p<0.05, **p<0.01, n=3). Figure 5 (C) and Figure 5(D) illustrate Gli36 and U251 cells infected with OV-Q1 or OVCDH1 at the MOI of 5 (1,000-fold higher than in A and B). 24, 48, 72 and 96 hours after infection the supernatant viral titers from each group were measured by plaque assay (*p<0.05, **p<0.01, n=3).

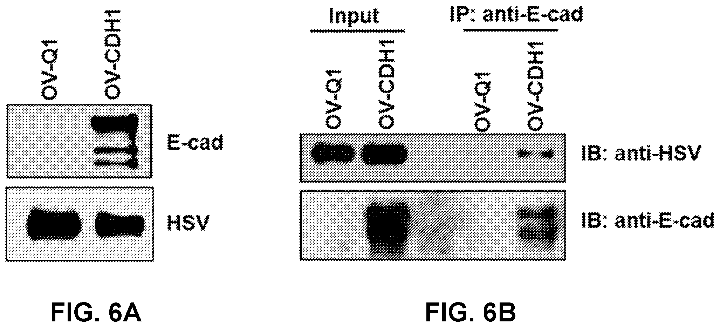

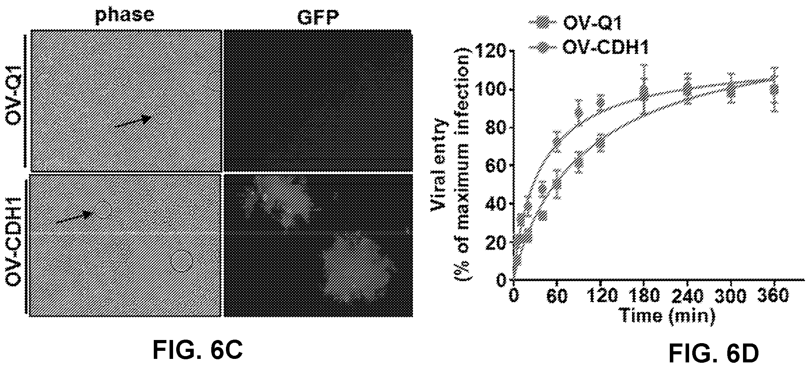

**Figures 6A-6D** **show viral membrane loaded E-cadherin and acceleration of viral entry of OV-CDH1.** Figure 6(A) illustrates immunoblotting assay of purified OV-CDH1 and OV-Q1 viral particles. Figure 6(B) illustrates immunoprecipitation (IP) assay with purified viral particles. Anti-E-cadherin-coated agarose beads (Invitrogen) were incubated with purified OV-CDH1 or OV-Q1 particles for 1 hour followed by washing (9 times). Pull-down lysates were detected by immunoblotting. Figure 6(C) illustrates images of agarose beads from Figure 6(B) that were added to the monolayers of Gli36 cells. The cells were imaged by microscopy 48 hours after adding the beads. Figure 6(D) illustrates viral entry speed assay of OV-CDH1 and OV-Q1. Gli36 cells were infected with OV-CDH1 or OV-Q1 at the MOI of 0.005. At 10, 20, 40, 60, 90, 120, 150, 180, 210, 240, 300 and 360 mins after infection, the infective media were removed and the cells were washed gently twice with PBS before overlay mediums were replaced. Plaque numbers were counted 48 hours after infection. The "viral entry-time" curves were established to evaluate the viral entry speed of OV-CDH1.

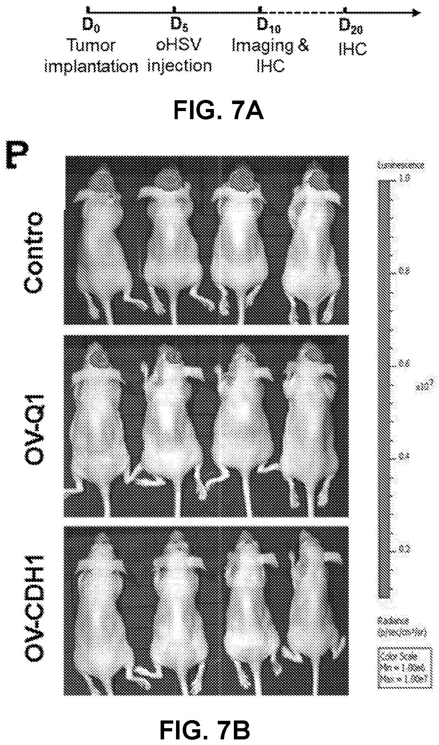

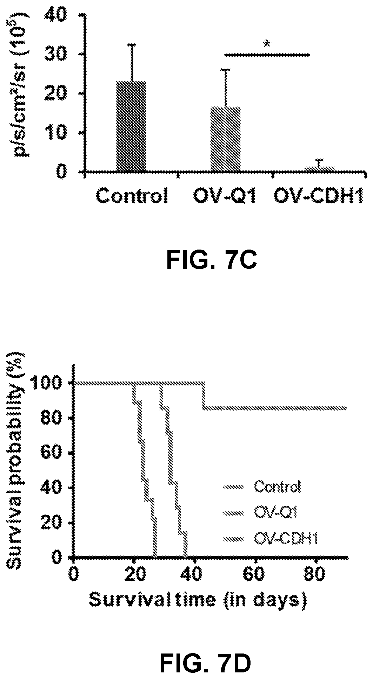

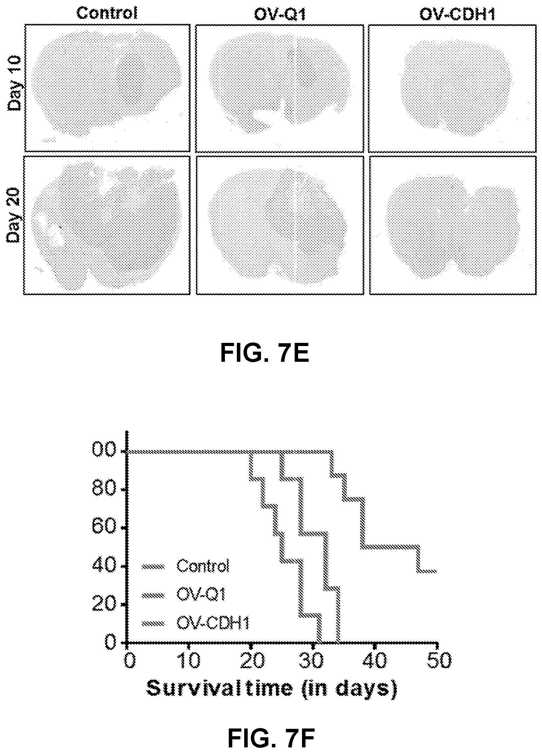

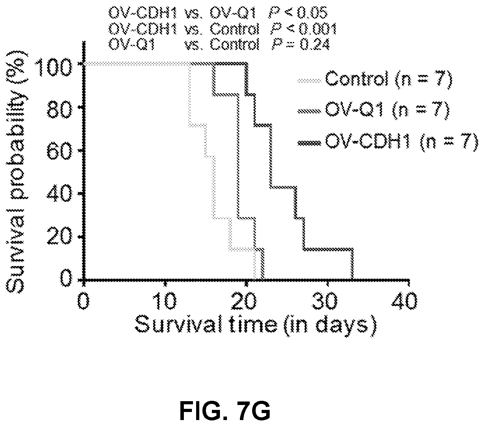

**Figures 7A-7G** **show that OV-CDH1 improves the efficacy on GBM virotherapy *in vivo.*** Figure 7(A) illustrates the experimental timeline for *in vivo* studies. A xenograft GBM mouse model and immunocompetent GBM mouse model were established by intracranially injecting 1 × 10⁵ GB30-FFL or Gl261Nectin cells to athymic nude mice or C57BL/6 mice. Five days after tumor cell implantation the mice were intratumorally injected with 2 × 10 10⁵ PFU of OVCDH1, OV-Q1 or vehicle. Figures 7(B) & 7(C) show luciferase-based imaging for evaluating the progress of GBM 10 days after tumor implantation. Figure 7(D) illustrates survival curves of OV-CDH1, OV-Q1, and vehicle treated xenograft GBM mice. This model was established by injecting GB30-FFL cells into athymic nude mice. Figure 7(E) illustrates images of H & E staining of mouse brains fixed and paraffin-embedded 10 days and 20 days after tumor implantation. Brain sections were cut through the oHSV injection site. Figure 7(F) illustrates survival curves of OV-CDH1, OV-Q1, and vehicle treated immunocompetent GBM mice. This mouse model was established by injecting Gl261Nectin cells into C57BL/6 mice. Figure 7(G) illustrates survival curves of Gl261N4 tumor-bearing C57BL/6 immunocompetent mice treated with vehicle control, OV-Q1, or OV-CDH1 as indicated in **(a).**

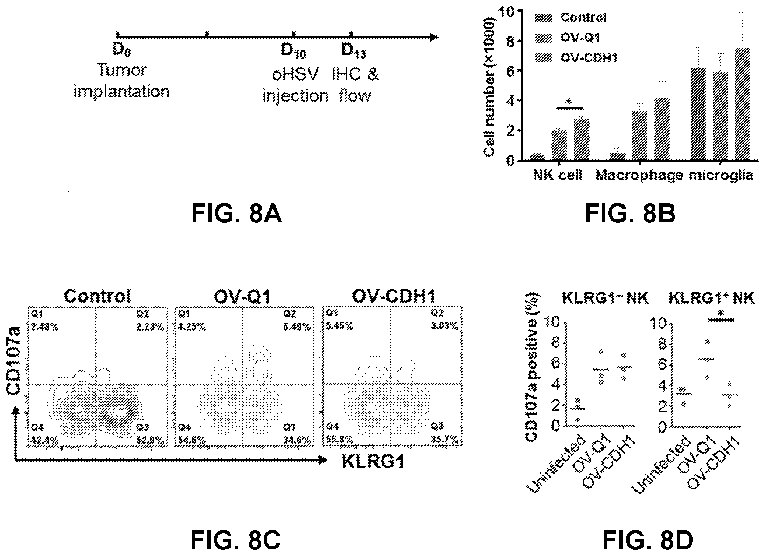

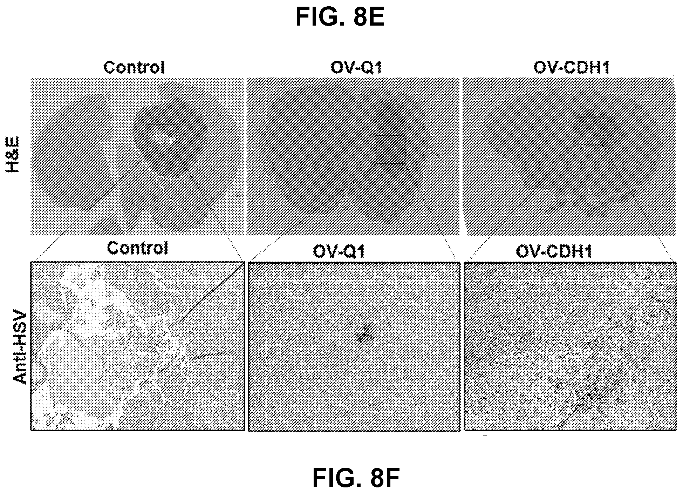

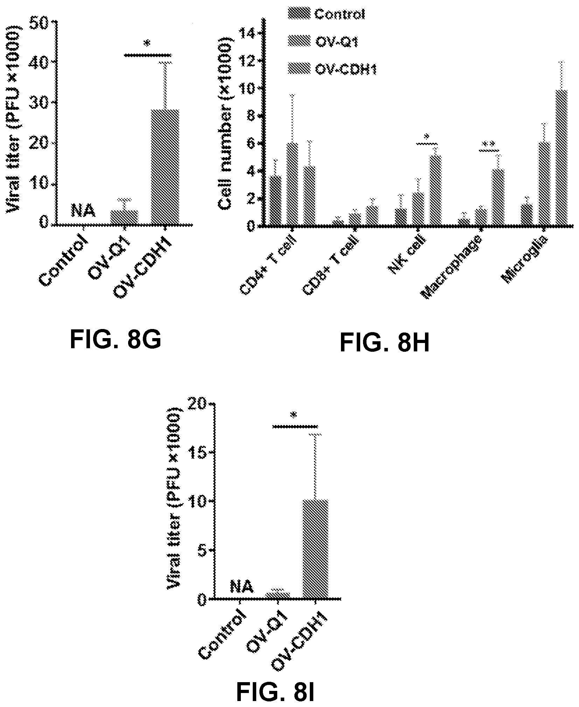

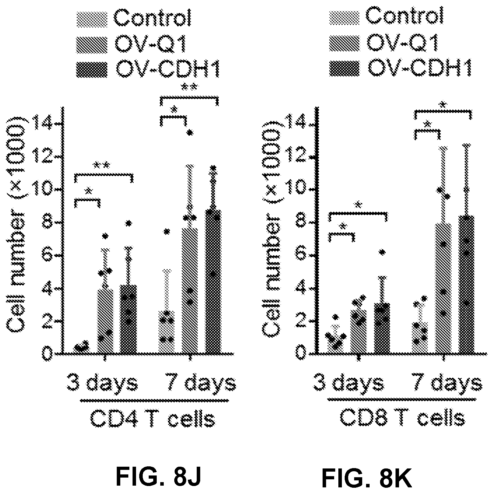

**Figures 8A-8K** **show that OV-CDH1 treatment improved immune cell infiltration and enhanced viral spread and oncolysis.** Xenograft and immunocompetent GBM mouse models were used to study the *in vivo* character of OV-CDH1. Figure 8(A) illustrates the experimental timeline for the *in vivo* study. Xenograft GBM mice were established by intracranially injecting 1 × 10⁵ GBM30 cells into athymic nude mice. 10 days after tumor implantation, mice were intratumorally injected with 2 × 10⁵ PFU of OV-Q1, OV-CDH1 or vehicle. 3 days after oHSV treatment, mice were euthanized, the brains were harvested. Figure 8(B) shows flow cytometry assay results for experimental mouse brain cells isolated 3 days after viral injection. NK cells (CD3⁻NKp46⁺ lymphocyte), macrophages (CD115⁺CD45^{high}CD11b⁺) and microglia (CD115⁺CD45^{low}CD11b⁺) were gated to evaluate immune cell recruitment. Figures 8(C) and 8(D) illustrate flow cytometry assay results of CD107a performed to evaluate the activity of NK cells. Figure 8(E) illustrates H&E staining of brain sections cut through the viral injection site. Figure 8(F) illustrates anti-HSV-1 IHC staining. Immunocompetent GBM mice were established by intracranially injecting 1 × 10⁵ Gl261N4 cells into C57BL/6 mice. 10 days after tumor implantation, mice were intratumorally injected with 2 × 10⁵ PFU of OV-Q1, OV-CDH1 or vehicle. 3 days after oHSV treatment, mice were euthanized, the brains were harvested. Figure 8G shows HSV genome copy numbers in the nude mouse brains that were measured by real time PCR to evaluate the viral production. Virus titers in the brains of the OV-treated nude mice were measured by qPCR with primers targeting HSV-1 gD gene 3 days after OV injection (**P* < 0.05, *n =* 3). Figure 8H shows immune cell infiltrations in these C57BL/6 mouse brains were measured by flow cytometry. Figure 8I shows HSV genome copy numbers in these C57BL/6 mouse brains were measured by real time PCR with the primers targeting HSV-1 gD gene 3 days after OV injection (**P* < 0.05, *n* = 3) to evaluate virus production. The intracranial infiltrations of CD4 T-cells (CD3⁺CD4⁺) (Figure 8J) and CD8 T-cells (CD3⁺CD8⁺) (Figure 8K) in Gl261N4-bearing mice measured by flow cytometry 3 and 7 days after viral injection (**P* < 0.05, ***P* < 0.01, ****P* <0.001, *n* = 6).

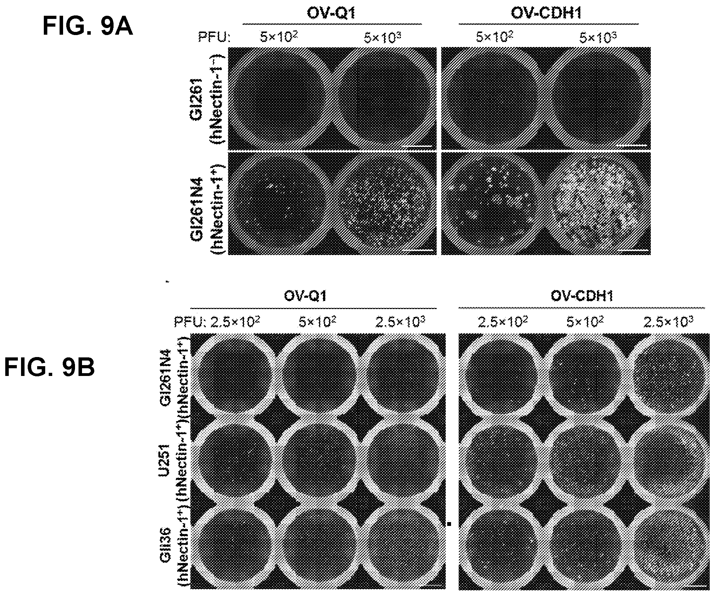

**Figures 9A and 9B** **show slow susceptibility of Gl261Nectin cells to oHSV infection.** Figure 9(A) illustrates Gl261 and Gl261N4 cells were infected with 5×10² and 5×10³ PFU of OV-Q1 or OV-CDH1. 48 hpi, the cells were imaged to detect GFP expression in virally infected cells with a florescence microscope to evaluate cell susceptibility to oHSV infection. Scale bar, 5000 µm. Figure 9(B) illustrates monolayers of Gl261Nectin, U251 and Gli36 cells were infected with 2.5×10², 5×10² and 2.5×10³ PFU of OV-Q1 or OV-CDH1. 36 hour after infection, the cells were observed and imaged by florescence microscopy.

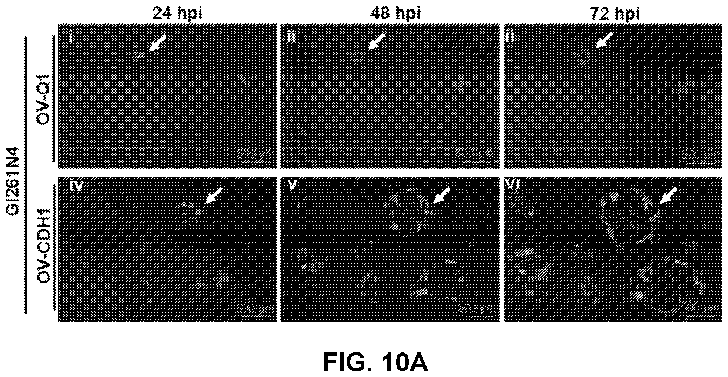

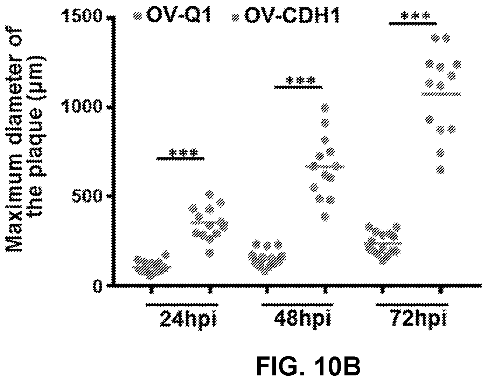

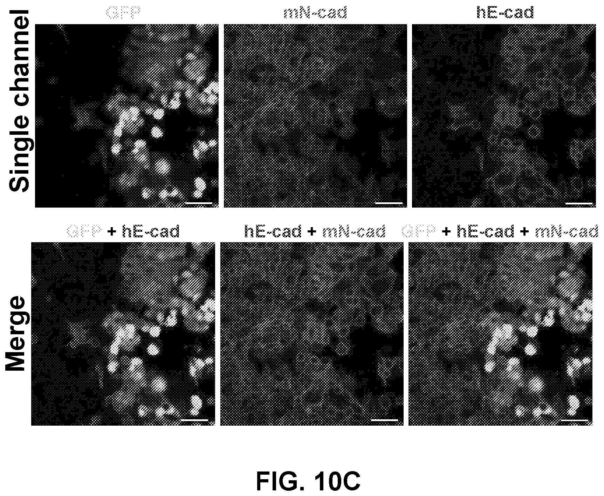

**Figures 10A-10C** **show enhanced viral spread of OV-CDH1 in Gl261Nectin cells.** Figure 10(A) illustrates monolayers of Gl261Nectin cells infected with OV-Q1 or OV-CDH1 at the MOI of 0.005. The cells were imaged by microscopy 24, 48 and 72 hours after infection. Each row shows the same vision at different times. The white arrows in each row point out the same plaque. Figure 10(B) illustrates plaque sizes from 3 randomly selected views from different groups (p<0.001, n=3). Figure 10(C) illustrates that Gl261N4 cells were infected with OV-CDH1. At 48 hpi, the cells were blocked by incubation with anti-E-cadherin or anti-N-cadherin followed by florescence secondary antibody staining. Cells were then observed under a confocal microscope.

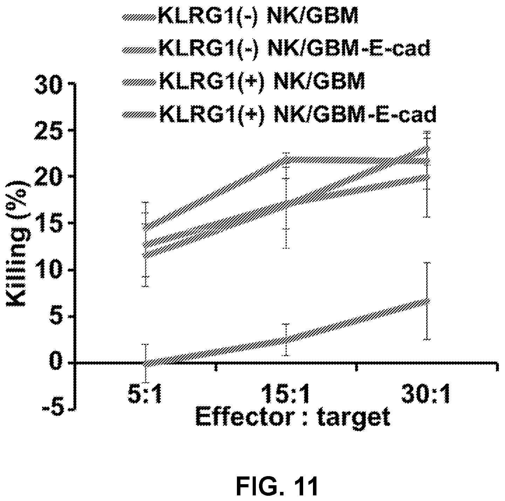

**Figure 11** **is a graph illustrating that Gli36 GBM cells ectopically overexpressing E-cad are less sensitive to KLRG⁺ NK cell cytotoxicity.** GLI36 GBM cells were generated and investigated for cytotoxic activity of KLRG⁺ and KLRG⁻ NK cells against these cells and control cells with an empty vector.

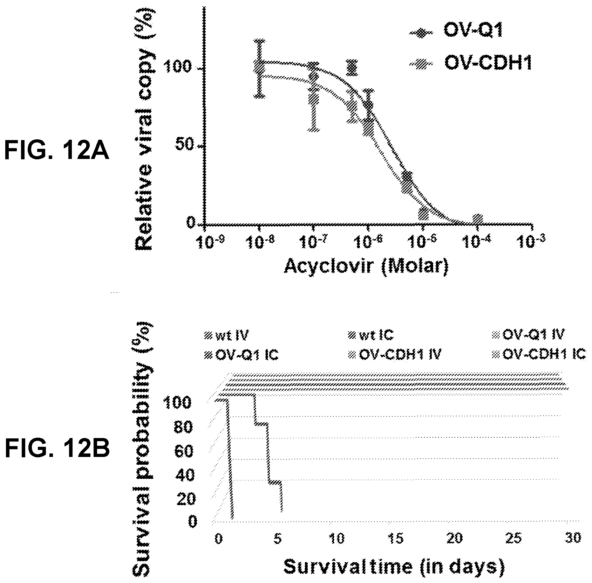

**Figures 12A-12B** **illustrate the safety test for OV-CDH1.** Figure 12(A) illustrates that Gli36 cells were infected with OV-Q1 or OV-CDH1 at a MOI of 5. Two hours after infection, the infection media were replaced with fresh media with increasing concentrations of Acyclovir. Forty-eight hours after infection, the cells were harvested for DNA extraction. The HSV-1 genome copy numbers in OV-Q1 and OV-CDH1 infected cells were measured by real-time PCR with primers and a probe against gD. The copy number vs. dose curves were established to evaluate the inhibitory effect of Acyclovir against oHSV replication (n = 3). Figure 12(B) illustrates survival curves of BLAB/c mice treated with wild-type HSV-1 (F strain), OV-Q1 or OV-CDH1 at the dose of 1×10⁶ via i.c. or i.v injections.

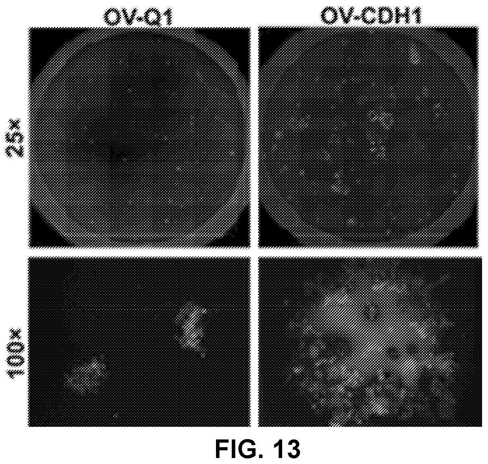

**Figure 13** **shows breast cancer cells (MDA-MB-468) infected with OV-Q1 or OV-CDH1 at the MOI of 0.005.** Human breast cancer MDA-MB-468 cells were seeded in 24 well plates. When the cells were 95% confluence, cells were infected with OV-Q1 or OV-CDH1 at the MOI of 0.005. 2 hours after infection the viral medium were replaced with fresh medium. 48 hours after infection, the plaques formed by different viruses were imaged with florescence microscope. Images were taken at 48 hours after infection.

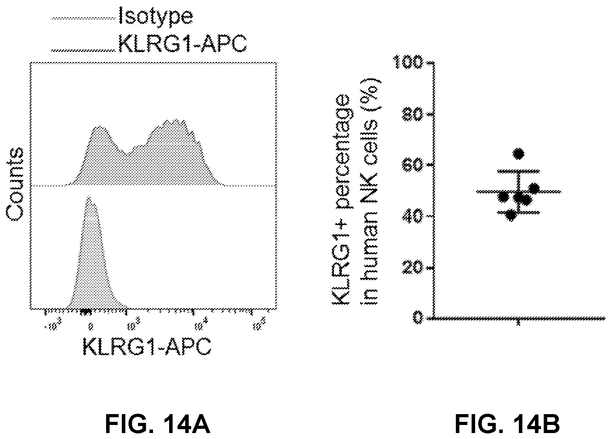

**Figures 14A-14B** **show KLRG1 expression in human NK cells.** For Figure 14A human NK cells were freshly isolated from the heathy donor and KRLG1 expression tested by flow cytometry. In Figure 14B, NK cell KLRG1 positive ratios of 6 different donors were used to show a normal range.

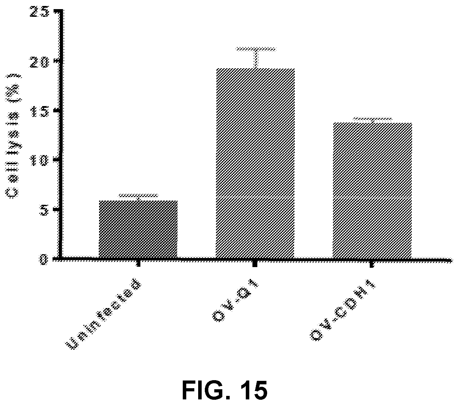

**Figure 15** **shows mouse NK cell cytotoxicity against OV-CDH1 infected cells measured by Cr⁵¹ release assay.** OV-Q1, OV-CDH1 or uninfected Gl261N4 cells were used as target cells. 8 hours after infection the cells were labeled with Cr⁵¹ for 1 hour. The labeled target cells were co-cultured with mouse NK cells that freshly isolated from C57BL/6 mice at the effector/target ratio of 35:1 for 4 hours. The Cr⁵¹ releases were measured to evaluated the cytotoxicity.

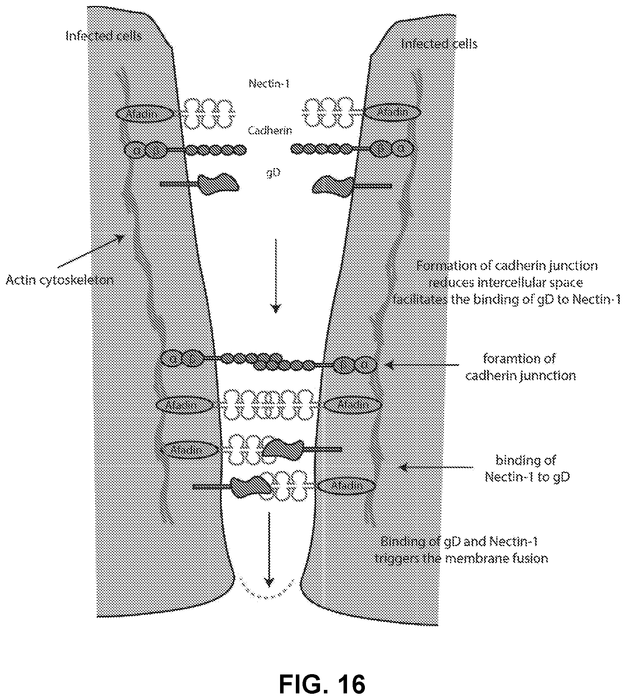

**Figure 16** **illustrates a proposed mechanism for OV-CDH1 to enhance viral spread.** OV-CDH1 infection introduces high level surface expression of E-cadherin to the infected cells. Cadherin interaction/binding shortens the intercellular distance and facilitates the binding of Nectin-1 to gD that triggers the membrane fusion between neighboring cells and results in the cell-to-cell infection of OV-CDH1.

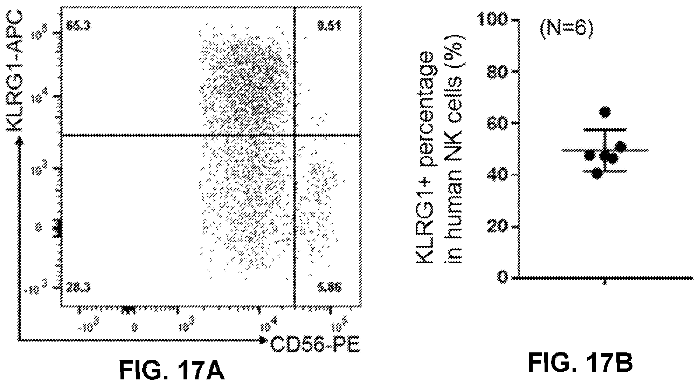

**Figures 17A-17B** **illustrate KLRG1 expression in human NK cells.** Figure 17(A) illustrates freshly isolated human NK cells were stained with anti-KLRG1-APC and anti-CD56-PE and analyzed with flow cytometry. Figure 17(B) illustrates the proportions of KLRG1⁺ and KLRG1⁻ of NK cells in peripheral blood assessed in 6 different healthy donors.

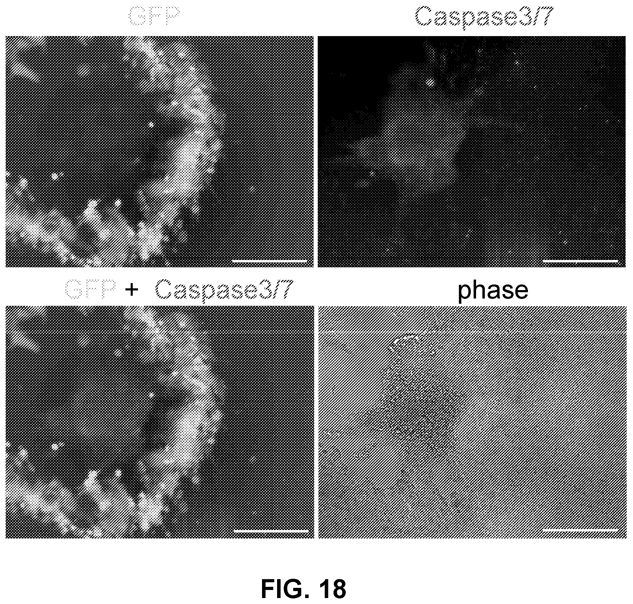

**Figure 18** **illustrates casepase-3/7 staining of OV-CDH1-infected U251 cells.** OV-CDH1 infected U251 cells were stained with Caspase-3/7 florescence dye and imaged at 48 hpi with a microscope. Scale bar, 150 µm. This experiment was performed in three technical replicates.

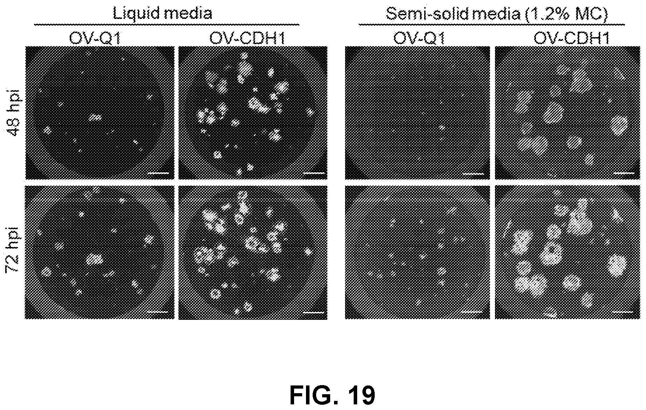

**Figure 19** **illustrates plaque-forming assay with liquid and semi-solid media.** Intratumoral viral spread environment was mimicked with semi-solid media containing 1.2% methylcellulose. Monolayers of U251 cells in 96-well plates were infected with OV-Q1 and OV-CDH. Two hours after infection, the infection media were replaced with fresh or semi-solid media containing 1.2% methylcellulose. The cells were then imaged at 48 and 72 hpi with a microscope. Scale bar, 1000 µm. This experiment was performed in three technical replicates.

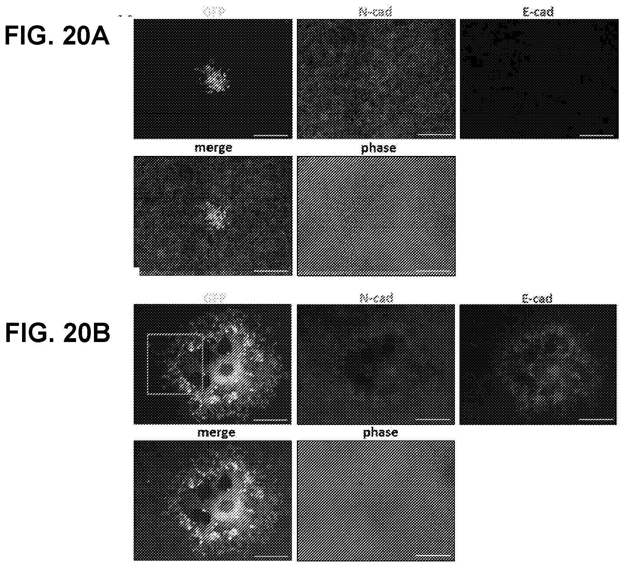

**Figures 20A-20B** **illustrate co-localization of human E-cadherin and human N-cadherin in OV-CDH1 infected human GBM cells.** U251 cells were infected with OV-Q1 (Figure 20(A)) or OV-CDH1 (Figure 20(B)). immunofluorescence staining process was performed with the infected cells using anti-E-cadherin and anti-N-cadherin at 48 hpi. Cells were then imaged using a microscope. The square indicates location of the image shown in Figure 5C. All images were taken using a high-resolution confocal microscope. Scale bar, 150 µm. This experiment was performed in three technical replicates.

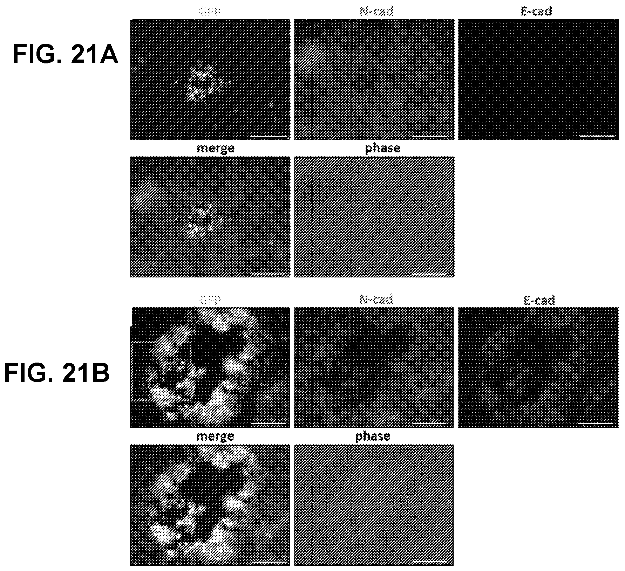

**Figures 21A-21B** **illustrate co-localization of human E-cadherin and murine N-cadherin in OV-CDH1 infected murine GBM cells.** Gl261N4 cells were infected with OV-Q1 Figure 21(A) or OV-CDH1 Figure 21(B). Immunofluorescence staining was performed on the infected cells using anti-E-cadherin and anti-N-cadherin antibodies at 48 hpi. The cells were then imaged using a microscope. The imaged were taken using a high-resolution confocal microscope. Scale bar, 150 µm. This experiment was repeated for three times.

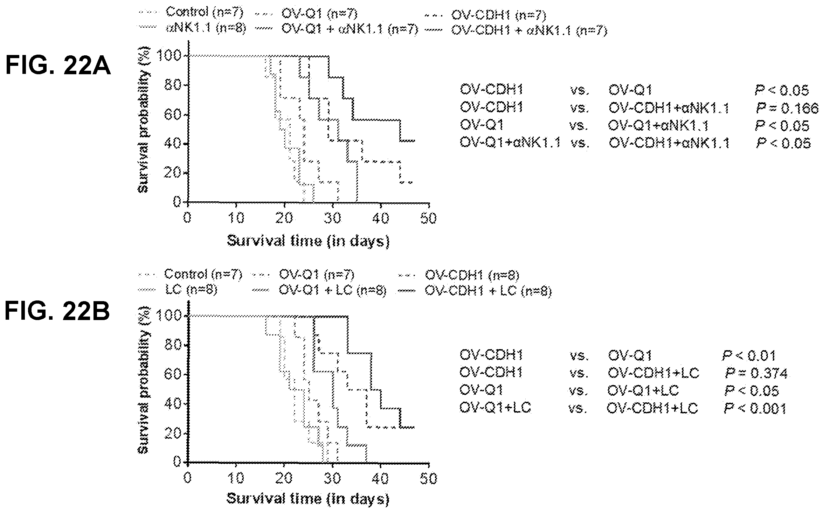

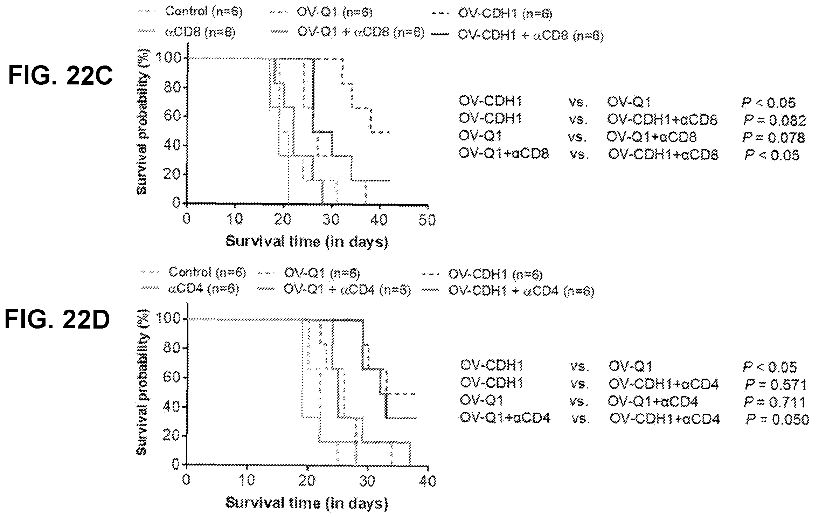

**Figures 22A-22D** **illustrate immune cell contribution in the OV-CDHl GBM therapy.** Figure 22(A) illustrates survival of Gl261N4 tumor-bearing C57BL/6 immunocompetent mice treated with vehicle control, OV-Ql, or OV-CDHl after NK cell depletion. The hazard ratio of OV-CDH1 to OV-Q1 in the presence vs. absence of NK cells is 0.17 vs. 0.32. Figure 22(B) illustrates survival of Gl261N4 tumor-bearing C57BL/6 immunocompetent mice treated with vehicle control, OV-Ql, or OV-CDHl after macrophage depletion. Figure 22(C) illustrates survival of Gl261N4 tumor-bearing C57BL/6 immunocompetent mice treated with vehicle control, OV-Ql, or OV-CDHl after CD8 T cell depletion. Figure 22(D) illustrates survival of Gl261N4 tumor-bearing C57BL/6 immunocompetent mice treated with vehicle control, OV-Q1, or OV-CDHl after CD4 T cell depletion.

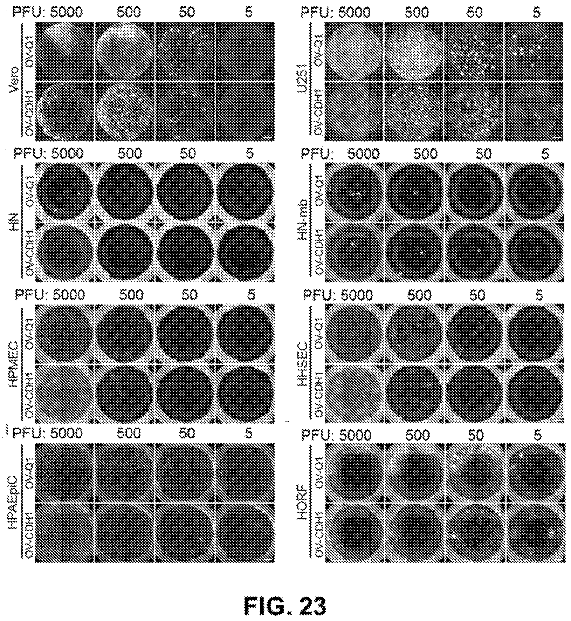

**Figure 23** **illustrates the tropism test for OV-CDHl.** Plaque forming assays were performed with 6 different types of human primary cells including oral fibroblasts (HorF), pulmonary microvascular endothelial cells (HPMEC), hepatic sinusoidal endothelial cells (HHSEC), pulmonary alveolar epithelial cells (HPAEpiC), neurons (HN), and neurons-midbrain (HN-mb). Vero and U251 cells were also included as control. At 48 hpi the cells were imaged under a microscope. Scale bar, 1000 µm.

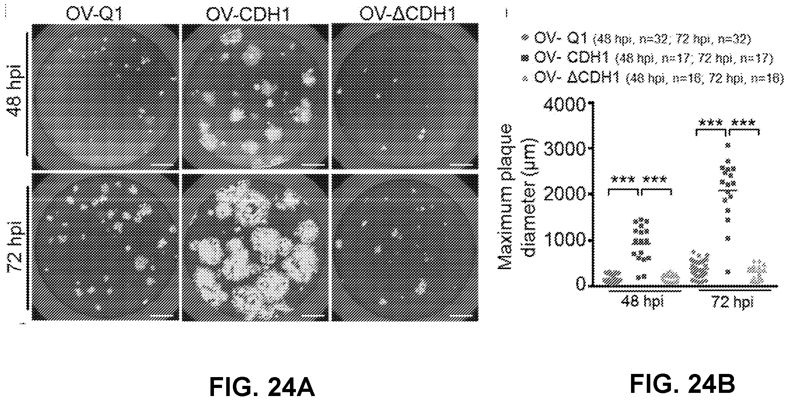

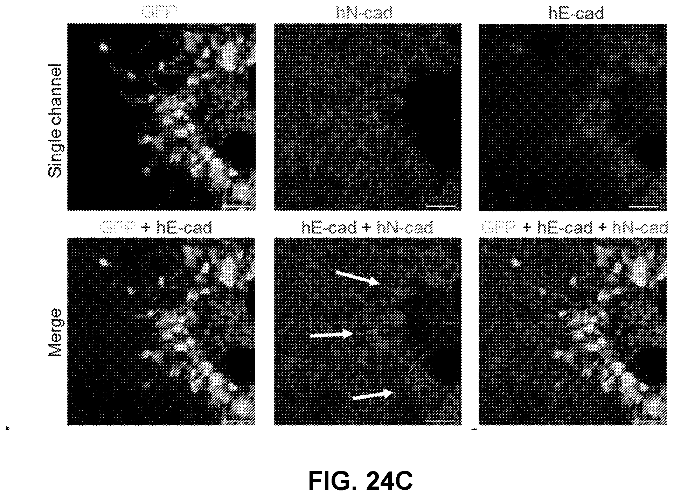

**Figures 24A-24C** illustrate cadherin interaction facilitates cell-to-cell infection by OV-CDHI. Figure 24(A) illustrates monolayers ofU251 cells in 96-well plates were infected with OV-Ql, OV-CDH1 and OV-11CDH1. Two hours after infection the infection media were replaced with semi-solid media containing 1.2 % methylcellulose. The cells were then imaged at 48 and 72 hpi with a florescent microscope. Scale bar, 1000 µm. Figure 24(B) illustrates the quantification of plaque diameter in (a) *(***P* < 0.001, sample sizes were indicated in the figures). Figure 24(C) illustrates that U25 l cells were infected with OV-CDHI. At 48 hpi, the cells were blocked and incubated with anti-E-cadherin and anti-N-cadherin, and followed by florescence secondary antibody staining. Cells were then observed under a confocal microscope. Scale bar, 50 µm.

### DETAILED DESCRIPTION

The disclosure provides oncolytic herpes simplex virus comprising a heterologous gene encoding E-cadherin. The disclosure further provides pharmaceutical
compositions that comprise such oncolytic herpes virus, and methods of treatment using such oncolytic herpes virus.

### Definitions

As utilized in accordance with the present disclosure, the following terms, unless otherwise indicated, shall be understood to have the following meanings. Unless otherwise required by context, singular terms shall include pluralities and plural terms shall include the singular.

The term "gene" is well understood in the art and is a polynucleotide encoding a polypeptide. In addition to the polypeptide coding regions, a gene can include noncoding regions including, but not limited to, introns, transcribed but untranslated segments, and regulatory elements upstream and downstream of the coding segments.

As used herein the terms "polypeptide", "peptide" and "protein" are used interchangeably to refer to polymers of amino acids of any length, with the understanding that the art recognizes pepitdes to generally be smaller than proteins and comprising less than 90-100 amino acids. These terms also include proteins that are post-translationally modified through reactions that include glycosylation, acetylation and phosphorylation.

As used herein the term "human cadherin (CDH1) gene" is Homo sapiens cadherin 1 (CDH1). In particular embodiments, the CDH1 is a transcript variant 1, mRNA, NCBI Reference Sequence: NM_004360.4 (127.2775) (SEQ ID NO: 1).

As used herein the term "ICP6 gene" refers to a gene encoding infected cell protein 6 (ICP6). ICP6 is the large subunit of ribonucleotide reductase (RR) and a key enzyme for nucleotide metabolism and viral DNA synthesis in non-dividing cells. In particular embodiments, the ICP6 gene is SEQ ID NO: 2.

As used herein the term "γ34.5 gene" refers to a gene related to pathogenicity of HSV-1 and a virus mutant obtained by deleting this gene has markedly reduced virus replication ability in normal cells. In particular embodiments, the γ34.5 gene is SEQ ID NO: 3.

As used herein, the term "inactivating mutation" indicates a mutation or alteration to a gene wherein the expression of that gene is significantly decreased, or wherein the gene product is rendered nonfunctional, or its ability to function is significantly decreased.

As used herein the term "inactivating deletion" refers to a deletion of the whole or portion of the gene or suppression of expression of the gene through substitution of some bases, modification, insertion of an unnecessary sequence or the like.

A "promoter" is generally a sequence or sequences of DNA that function when in a relatively fixed location in regard to the transcription start site. A "promoter" contains core elements required for basic interaction of RNA polymerase and transcription factors and can contain upstream elements and response elements.

As used herein, the term "heterologous gene" refers to a gene derived from a species other than a recombinant host including virus.

As used herein the term "operably linked," refers to an arrangement of flanking sequences wherein the flanking sequences so described are configured or assembled so as to perform their usual function. Thus, a flanking sequence operably linked to a coding sequence may be capable of effecting the replication, transcription, and/or translation of the coding sequence. For example, a coding sequence is operably linked to a promoter when the promoter is capable of directing transcription of that coding sequence.

As used herein the term "subject" refers to any individual who is the target of administration or treatment. The subject can be a vertebrate, for example, a mammal. Thus, the subject can be a human or veterinary patient. The term "patient" refers to a subject under the treatment of a clinician, e.g., physician.

As used herein a "disorder" is any condition that would benefit from treatment using the recombinant viruses of the disclosure. "Disorder" and "condition" are used interchangeably herein.

As used herein the terms "treatment" or "treat," refer to both therapeutic treatment and prophylactic or preventative measures. Those in need of treatment include those having the disorder as well as those prone to have the disorder or those in which the disorder is to be prevented.

As used herein the terms "pharmaceutical composition" or "therapeutic composition," refer to a compound or composition capable of inducing a desired therapeutic effect when properly administered to a patient.

The term "pharmaceutically acceptable carrier" or "physiologically acceptable carrier," as used herein, refers to one or more formulation materials suitable for accomplishing or enhancing the delivery of the recombinant viruses of the disclosure.

The term "therapeutically effective amount" when used in reference to a pharmaceutical composition comprising one or more recombinant viruses of the disclosure refers to an amount or dosage sufficient to produce a desired therapeutic result. More specifically, a therapeutically effective amount is an amount of one or more recombinant viruses of the disclosure sufficient to inhibit, for some period of time, one or more of the clinically defined pathological processes associated with the condition being treated. The therapeutically effective amount may vary depending on the specific recombinant virus that is being used, and also depends on a variety of factors and conditions related to the patient being treated and the severity of the disorder. The determination of a therapeutically effective amount of a given pharmaceutical composition is well within the ability of those of skill in the art.

### Recombinant Oncolytic Herpes Virus

Disclosed herein are recombinant oncolytic herpes viruses comprising a heterologous gene encoding E-cadherin. The disclosed recombinant viruses may be derived from several different types of herpes viruses. The Herpesviridae are a large family of DNA viruses that cause diseases in humans and animals. Herpes viruses all share a common structure and are composed of relatively large double-stranded, linear DNA genomes encoding 100-200 genes encased within an icosahedral protein cage called the capsid which is itself wrapped in a lipid bilayer membrane called the envelope. This particle is known as the virion. The large genome provides many non-essential sites for introducing one or more transgenes without inactivating the virus (e.g., without completely inhibiting infection or replication). However, it should be appreciated that virus vectors are preferably modified (e.g., replication conditional, attenuated) so that they do not have undesirable effects (e.g., killing normal cells or causing disease).

The disclosed recombinant herpes viruses may comprise any one of a number of mutations that affect expression of a viral gene. In most cases, a mutation is in a virulence-related gene that contributes to the pathogenicity of the virus to a host organism. The mutation may be a point mutation, a deletion, an inversion, or an insertion. Typically the mutation is an inactivating mutation or deletion.

Several types of replication-conditional herpes virus mutants have been developed. For example, one aspect involves viral mutants with defects in the function of a viral gene needed for nucleic acid metabolism, such as thymidine kinase (Martuza et al., 1991, Science 252:854-856), ribonucleotide reductase (RR) (Goldstein & Weller, 1988, J. Virol. 62:196-205; Boviatsis et al., 1994, Gene Ther. 1:323-331; Boviatsis et al., 1994, Cancer Res. 54:5745-5751; Mineta et al., 1994, Cancer Res. 54:3363-3366), or uracil-N-glycosylase (Pyles & Thompson, 1994, J. Virol. 68:4963-4972). Another aspect involves viral mutants with defects in the function of the γ-34.5 gene (Chambers et al., 1995, Proc. Natl. Acad. Sci. USA 92:1411-1415), which functions as a virulence factor by markedly enhancing the viral burst size of infected cells through suppression of shutoff of host protein synthesis (Chou et al., 1990, Science 250:1262-1266; Chou & Roizman, 1992, Proc. Natl. Acad. Sci. USA 89:3266-3270). Other examples include G207 (Mineta et al., 1995, Nat. Med 1:938-943; U.S. Pat. No. 5,585,096, issued Dec. 17, 1996 to Martuza et al.), and MGH1 (Kramm et al., 1997, Hum. Gene Ther. 8:2057-2068), which possess deletions of both copies of γ-34.5 and an insertional mutation of RR.

Recombinant herpes viruses disclosed herein can involve viruses that are based on herpes viruses, such as herpes simplex viruses (HSV), for example, HSV-1 (*e.g.,* HSV-1 strain F or strain Patton) or HSV-2, that include an inactivating mutation in a virulence gene. In the case of herpes simplex viruses, this mutation can be an inactivating mutation in the γ-34.5 gene, which is the major HSV neurovirulence determinant.

In particular embodiments, the viruses disclosed herein can include a mutation or modification that is made to prevent reversion of the virus to wild type. For example, the virus can include a mutation in the ICP6 gene, which encodes the large subunit of ribonucleotide reductase.

The immune response plays both anti-tumor and anti-viral roles, which can alternately help and thwart treatment attempts. During the early stage of oHSV infection, the innate immune system primarily responds to the virus, killing the virally infected cells but also inhibiting oHSV viral reproduction. (Fulci et al., 2006, Proc. Natl. Acad. Sci. USA 103, 12873-12878; Breitbach et al., 2007, Molecular therapy : the Journal of the American Society of Gene Therapy 15, 1686-1693; Altomonte et al., 2009, Cancer gene therapy 16, 266-278; Alvarez-Breckenridge et al., 2012, Nat. Med. 18, 1827-1834). Over-rapid clearance of oHSV infection at this stage seriously impairs oncolytic efficacy of replicable oncolytic virus. However, at the late stage of oHSV infection, after infected tumor cells have been lysed by virus, the release of tumor antigens can trigger a significant antigen-specific anti-tumor adaptive immune response. Activating the adaptive immune response in this stage may help eliminate additional tumor cells otherwise missed by the virus (*see,* Prestwich et al., 2008, Clinical Cancer Research 14, 7358-7366; Prestwich et al., 2009, Clinical Cancer Research 15, 4374-4381). As the first line of host antiviral defense, innate immunity responds quickly to the viral infection and plays a dominant role on clearing oHSV infection at the early stage of oHSV therapy. Within hours of oHSV administration, NK cells are activated and recruited to the viral injection site, causing an over-rapid clearance of oHSV infection and limiting the efficacy of oHSV therapy (Fulci et al., 2006, Proc. Natl. Acad. Sci. USA 103, 12873-12878; Altomonte et al., 2009, Cancer Gene Therapy 16, 266-278; MacTavish et al., 2010, PloS one 5, e14462; Alvarez-Breckenridge et al., 2012, Nat. Med. 18, 1827-1834; Alvarez-Breckenridge et al., 2012, J. Virol. 86, 4566-4577; Bridle et al., 2013, Molecular Therapy : The Journal of the American Society of Gene Therapy 21, 887-894; Han et al., 2015, Cancer Research 75, 5273-5282). When NK cells are inhibited by pre-treatments with NK blocking antibodies, TGF-beta, or histone deacetylase inhibitors before virus administration, oHSV virotherapy shows significantly better outcomes (Fulci et al., 2006, Proc. Natl. Acad. Sci. USA 103, 12873-12878; Altomonte et al., 2009, Cancer Gene Therapy 16, 266-278; MacTavish et al., 2010, PloS one 5, e14462; Alvarez-Breckenridge et al., 2012, Nat. Med. 18, 1827-1834; Alvarez-Breckenridge et al., 2012, J. Virol. 86, 4566-4577; Bridle et al., 2013, Molecular Therapy : The Journal of the American Society of Gene Therapy 21, 887-894; Han et al., 2015, Cancer Research 75, 5273-5282). Systematic inhibition of NK cells, however, causes inevitably reduced antitumor activity over the course of treatment.

In particular embodiments, provided herein are recombinant oncolytic herpes simplex virus comprising a heterologous gene encoding a human E-cadherin gene (CDH1) driven by the HSV-1 immediate early gene promoter, pIE4/5 (referred to herein as OV-CDH1) to specifically inhibit antiviral activity while maintaining the antitumor activity of NK cells.

E-cadherin inhibits cytotoxicity of NK cells by binding to its receptor KLRG1 on NK cells, preventing lysis of E-cadherin-expressing cells by KLRG1⁺ NK cells (Ito et al., 2006, J. Exp. Med. 203, 289-295; Schwartzkopff et al., 2007, J. Immunol. 179, 1022-1029; Li et al., 2009, Immunity 31, 35-46).

The recombinant herpes viruses disclosed herein advantageously provide for inhibition of antiviral activity of natural killer (NK) cells through the KLRG1-E-cadherin inhibitory axis. This inhibition provides more time and space for OV-CDH1 to complete viral reproduction and initiate secondary infection of additional tumor cells. Additionally, OV-CDH1 can stimulate considerable adaptive immune response to suppress tumor growth and progression.

oHSV has two different strategies for viral spread. One is dependent on the viral particles, which can diffuse, bind, and infect distant target cells. This kind of viral spread can easily occur in cell culture due to liquid media conditions. In the context of a solid tumor such as GBM in patients, the other viral spread strategy, cell-to-cell infection, permits additional viral spread. Cell-to-cell infection occurs between tightly jointed neighboring cells and results in direct transfer of the virus from an infected cell to an uninfected cell. However, inside a solid tumor, many factors such as the extracellular matrix, areas of fibrosis and necrosis, and surrounding normal cells in the tumor bed negatively affect viral particle diffusion as well as cell-to-cell infection oHSV. Ultimately, viral spread for most oHSV is quite limited inside a solid tumor. As a well-known calcium-dependent cell-cell adhesion molecule, E-cadherin plays an important role in cell adhesion and forming adherens junctions. (Christofori et al., 1999, Trends in Biochemical Sciences 24, 73-76) which can *inter alia* facilitate viral spread.

The recombinant viruses disclosed herein significantly enhance cell-to-cell infection and improve viral entry. These advantages both profit from enhancement of HSV-dependent lipid membrane fusion by E-cadherin. HSV-dependent membrane fusion begins with binding of gD to its specific receptors (nectin-1 or HVEM). The binding of gD subsequently induces conformational changes in gH/gL and gB; finally, gB inserts its fusion loops into the opposing lipid membrane and completes membrane fusion (Eisenberg et al., 2012, Viruses 4, 800-832). Importantly, nectin-1 is a calcium-independent cell-cell adhesion molecule that cooperates with cadherins to forming of adherens (Nakanishi et al., 2004, Biological Chemistry 385, 885-892). Cadherin adhesion can recruit nectin-1 to stabilize the adherens junctions (Takahashi et al., 1999, J. Cell Biol. 145, 539-549).

Examples of non-tissue specific promoters that can be used in the disclosed oHSV include the early Cytomegalovirus (CMV) promoter (U.S. Pat. No. 4,168,062) and the Rous Sarcoma Virus promoter (Norton et al., 1985, Molec. Cell. Biol. 5: 281). Also, HSV promoters, such as HSV-1 IE and IE 4/5 promoters, can be used.

Examples of promoters that function specifically in tumor cells include but are not limited to the stromelysin 3 promoter, which is specific for breast cancer cells (Basset et al., 1990, Nature 348: 699); the surfactant protein A promoter, which is specific for non-small cell lung cancer cells (Smith et al., 1994, Hum. Gene Ther. 5: 29-35); the secretory leukoprotease inhibitor (SLPI) promoter, which is specific for SLPI-expressing carcinomas (Garver et al., 1994, Gene Ther. 1: 46-50); the tyrosinase promoter, which is specific for melanoma cells (Vile et al., 1994, Gene Ther. 1: 307; WO 94/16557); the stress inducible grp78/BiP promoter, which is specific for fibrosarcoma/tumorigenic cells (Gazit et al., 1995, Cancer Res. 55 (8): 1660); the AP2 adipose enhancer, which is specific for adipocytes (Graves, 1992, J. Cell. Biochem. 49: 219); the a-1 antitrypsin transthyretin promoter, which is specific for hepatocytes (Grayson et al., 1988, Science 239: 786); the interleukin-10 promoter, which is specific for glioblastoma multiform cells (Nitta et al., 1994, Brain Res. 649: 122) as well as several immune cells including NK cells and monocytes; the c-erbB-2 promoter, which is specific for pancreatic, breast, gastric, ovarian, and non-small cell lung cells (Harris et al., 1994, Gene Ther. 1: 170); the a-B-crystallin/heat shock protein 27 promoter, which is specific for brain tumor cells (Aoyama et al., 1993, Int. J. Cancer 55: 760); the basic fibroblast growth factor promoter, which is specific for glioma and meningioma cells (Shibata et al., 1991, Growth Fact. 4: 277); the epidermal growth factor receptor promoter, which is specific for squamous cell carcinoma, glioma, and breast tumor cells (Ishii et al., 1993, Proc. Natl. Acad. Sci. U.S.A. 90: 282); the mucin-like glycoprotein (DF3, MUC1) promoter, which is specific for breast carcinoma cells (Abe et al., 1993, Proc. Natl. Acad. Sci. U.S.A. 90: 282); the mtsl promoter, which is specific for metastatic tumors (Tulchinsky et al., 1992, Proc. Natl. Acad. Sci. U.S.A. 89: 9146); the NSE promoter, which is specific for small-cell lung cancer cells (Forss-Petter et al., 1990, Neuron 5: 187); the somatostatin receptor promoter, which is specific for small cell lung cancer cells (Bombardieri et al., 1995, Eur. J. Cancer 31A: 184, 1995; Koh et al., Int. J. Cancer 60: 843); the c-erbB-3 and c-erbB-2 promoters, which are specific for breast cancer cells (Quin et al., 1994, Histopathology 25: 247); the c-erbB4 promoter, which is specific for breast and gastric cancer cells (Rajkumar et al., 1994, Breast Cancer Res. Trends 29: 3); the thyroglobulin promoter, which is specific for thyroid carcinoma cells (Mariotti et al., 1995, J. Clin. Endocrinol. Meth. 80: 468); the a-fetoprotein promoter, which is specific for hepatoma cells (Zuibel et al., 1995, J. Cell. Phys. 162: 36); the villin promoter, which is specific for gastric cancer cells (Osborn et al., 1988, Virchows Arch. A. Pathol. Anat. Histopathol. 413: 303); and the albumin promoter, which is specific for hepatoma cells (Huber, 1991, Proc. Natl. Acad Sci. US.A. 88: 8099).

### Therapeutic Compositions Comprising Recombinant Viruses andAdministration Thereof

The disclosure further relates to pharmaceutical compositions and therapeutic uses of the recombinant viruses of the disclosure.

The therapeutic or pharmaceutical compositions disclosed herein can comprisea therapeutically effective amount of a recombinant oncolytic herpes simplex virus as disclosed herein in admixture with a pharmaceutically or physiologically acceptable formulation agent selected for suitability with the mode of administration. Acceptable formulation materials are preferably nontoxic to recipients at the dosages and concentrations to beemployed.

Acceptable formulation materials can be used to modify, maintain, or preserve, for example, the pH, osmolarity, viscosity, clarity, color, isotonicity, odor, sterility, stability, rate of dissolution or release, adsorption, or penetration of the composition. Acceptable formulation materials include, but are not limited to, amino acids (such as glycine, glutamine, asparagine, arginine, or lysine), antimicrobials, antioxidants (such as ascorbic acid, sodium sulfite, or sodium hydrogen-sulfite), buffers (such as borate, bicarbonate, Tris-HCl, citrates, phosphates, or other organic acids), bulking agents (such as mannitol or glycine), chelating agents (such as ethylenediamine tetraacetic acid (EDTA)), complexing agents (such as caffeine, polyvinylpyrrolidone, beta-cyclodextrin, or hydroxypropyl-beta-cyclodextrin), fillers, monosaccharides, disaccharides, and other carbohydrates (such as glucose, mannose,or dextrins), proteins (such as serum albumin, gelatin, or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as polysorbate 20 or polysorbate 80; triton;
tromethamine; lecithin; cholesterol or tyloxapal), stability enhancing agents (such as sucrose or sorbitol), tonicity enhancing agents (such as alkali metal halides - preferably sodium or potassium chloride - or mannitol sorbitol), delivery vehicles, diluents, excipients and/or pharmaceutical adjuvants (see, e.g., Remington's Pharmaceutical Sciences (18th Ed., AR. Gennaro, ed., Mack Publishing Company 1990), and subsequent editions of the same).

A skilled artisan can determine the optimal pharmaceutical composition comprising the recombinant viruses disclosed herein depending upon, for example, the intended route of administration, delivery format, and desired dosage.

The recombinant viruses disclosed herein can be administered in any suitable manner, such as intracranially, intravenously, subcutaneously, via injection or infusion, or in any other suitable manner that allows the viruses to enter the circulation. The preparation of such pharmaceutical compositions is within the knowledge of one of skill in the art

The pharmaceutical compositions of the disclosure can also be selected for parenteral delivery. Alternatively, the compositions can be selected for inhalation or for delivery through the digestive tract, such as orally. The preparation of such pharmaceutical compositions is within the knowledge of one of skill in the art.

Additional pharmaceutical compositions will be evident to those of skill in the art, including formulations involving sustained-delivery or controlled-delivery formulations. Techniques for formulating sustained-delivery or controlled-delivery formulations, using, for example, liposome carriers, bio-erodible microparticles or porous beads, and depot injections, are known to those of skill in the art.

In one embodiment, the disclosure provides a method for preventing and/or treating at least one disease, condition, or disorder that can be prevented or treated using the recombinant viruses disclosed herein. These methods include administering to a patient in need thereof a therapeutically or pharmaceutically effective amount of an oncolytic herpes simplex virus (HSV), comprising a heterologous gene encoding E-cadherin. In particular embodiments, the disorder is a tumor such as a solid tumor. In particular embodiments, the solid tumor is a glioblastoma, an ovarian tumor or a breast tumor.

The effective amount of a pharmaceutical composition as disclosed herein to be employed therapeutically will depend, for example, upon the therapeutic context and objectives.

One of skill in the art will appreciate that an appropriate dosage level for treatment will vary depending, in part, upon the molecule being delivered, the indication for which the composition is being used, the route of administration, and the size (body weight, body surface, or organ size) and condition (age and general health) of the patient.

Without limiting the disclosure, a number of embodiments of the disclosure are described below for purpose of illustration.

### EXAMPLES

The Examples that follow are illustrative of specific embodiments of the disclosure, and various uses thereof. They are set forth for explanatory purposes only, and should not be construed as limiting the scope of the invention in any way.

### Example 1. Materials and Methods

### Cells

Human GBM cell lines (U87, Gli36 and U251) and mouse GBM cells lines (Gl261 and Gl261Nectin) were maintained with DMEM media supplemented with 10% fetal bovine serum (FBS), penicillin (100 U/ml), and streptomycin (100 µg/ml). Gl261Nectin cells were modified to overexpress human Nectin-1 to be susceptible to oHSV infection. GBM30 cells are mesenchymal GBM stem-like cells derived from a patient and form neurosphere in the culture and have a greater potential of cancer cell initiation and repopulation (Mao et al., 2013, Proc. Natl. Acad. Sci. USA 110, 8644-8649). GBM30 models are expected to recapitulate the clinical patient GBM (Uchida et al., 2013, Molecular therapy: the journal of the American Society of Gene Therapy 21, 561-569). GBM30 spereroid cells were derived from a GBM patient and were modified to overexpress luciferase, named GBM-FFL, for *in vivo* imaging. GBM30 and GBM30-FFL cells were maintained as tumor spheres with neurobasal media supplemented with 2% B27 (Gibco), human epidermal growth factor (20 ng/ml), and basic fibroblast growth factor (20 ng/ml) in low-attachment cell culture flasks. Monkey kidney epithelial-derived Vero cells were maintained with the same media of glioblastoma cell lines and were used for viral propagation and plaque assay-based viral titration. To knock down N-cadherin expression in Gli36 cells, the cells were infected with lentivirus coding N-cadherin-shRNA (TRCN0000312701, Sigma). Human primary cells including oral fibroblasts, pulmonary microvascular endothelial cells, hepatic sinusoidal endothelial cells, pulmonary alveolar epithelial cells, neurons, and neurons-midbrain were purchased from ScienCell and cultured following manufacturer's instructions.

### Viruses

OV-CDH1 and OV-ΔCDH1 (also termed E-oHSV herein) was engineered by using fHsvQuik-1 system as previously described (Drees et al., 2005, Cell 123, 903-915). Briefly, the full-length coding sequence of human CDH1 gene (SEQ ID NO: 1) was cloned and inserted into pT-oriSIE4/5 following the HSV IE4/5 promoter to construct the pT-oriSIE4/5-CDH1. And then pT-oriSIE4/5 and pT-oriSIE4/5-CDH1 were used to recombine with fHsvQuik-1 to engineer OV-Q1 and OV-CDH1, respectively. To generate OV-ΔCDH1, the original E-cadherin extracellular domain was replaced with the extracellular domain of interleukin-2 receptor alpha chain (IL-2Rα). The cytoplasmic domain of E-cadherin remained intact (Gottardi et al., 2001, J. Cell Biol. 153, 1049-1060). Viruses were propagated and titrated with Vero cells. Virus titration was performed with plaque assays. Briefly, monolayer Vero cells were infected with gradient diluted viral solutions. Two hours after infection the infection media were replaced with overlay media containing 1.2 % methylcellulose. The GFP-positive plaques were counted with a Zeiss florescence microscope (AXIO observer.Z1) 2 days after infection for calculating the virus titer.

### NK cell cytotoxicity assay

U87, Gli36, U251 and GBM30 cells were infected with OV-Q1, OV-CDH1 or OV-ΔCDH1 at the MOI of 3.0. Eight hours after infection the infected cells were used as target cells. Target cells were labeled with ⁵¹Cr for 1 hour, and then co-cultured with bulk human primary NK cells or sorted KLRG1⁺ and KLRG1⁻ NK cells at different effector-target ratios at 37° C for 4 hours. Release of ⁵¹Cr was measured with a Beckman Liquid Scintillation Counter LS-6500 (Beckman Coulter, Fullerton, CA). Target cells incubated in complete media or 1% SDS mediawere used for spontaneous or maximal ⁵¹Cr release control, respectively. The cell lysis percentages were calculated using the standard formula: 100 × (cpm experimental release - cpm spontaneous release) / (cpm maximal release - cpm spontaneous release).

### Viral spread/production assays

For viral spread assays, monolayers of U251, Gli36 and Gl261Nectin cells were infected with OV-Q1, OV-CDH1 or OV-ΔCDH1 at the MOI of 0.005. Two hours after infection, the infection media were replaced with fresh media or semi-solid media (1.2% methylcellulose). To investigate the role of E-cadherin in enhancing virus spread of OV-CDH1, the E-cadherin neutralizing antibody (clone DECMA-1) (100 ng/ml) or the gD neutralizing antibody (clone B007 LP2) (10 ng/ml) was included in the media. For the plaque forming assays, cell membrane and nuclei were labeled using Celltracker^{™} Deep Red and DAPI at 24 hpi. Apoptosis was assessed using CellEvent^{™} Caspase-3/7 Red staining at 24 hpi. The plaques in each group were imaged with fluorescent microscopy at 24, 48, and 72 hpi. Videos of plaque formation were recorded with the Zeiss florescence microscope (AXIO observer.Z1) system from 24 to 72 hpi.

For viral production assays, monolayers of U251 and Gli36 cells were infected with OVQ1 or OV-CDH1 at the MOIs of 5 or 0.005. Two hours after infection, the infection media were replaced with fresh media. The supernatants from each group were then harvested at 24, 48, 72, and 96 hpi and titrated by plaque assays.

### Flow cytometry

To detect surface expression assay of human E-cadherin, OV-CDH1 or OV-Q1 infected glioblastoma cells were stained with anti-hE-cadherin-APC antibody (R&D, FAB18381) for 30 min in the Ga²⁺ conditional staining buffer. To detect surface expression of human mutant E-cadherin (E-cad/IL-2Rα/), an anti-CD25-APC antibody was used. For the CD107a staining assay, eight hours after infection, the OV-infected or uninfected glioblastoma cells were co-cultured with NK cells at the ratio of 1:1 with the presence of anti-107CDa-PE. One hour later, GolgiStop^{™} (BD Biosciences) was added. After 4 hours co-culture, the cells were harvested and stained with anti-CD56-APC-H7, anti-CD3-V450 and anti-KLRG1-APC for 30 minutes. All flow cytometry data were collected using an LSR II cytometer (BD Biosciences).

### Animal studies

Six- to eight-week-old female athymic nude mice and C57BL/6 mice were purchased from Jackson Laboratories (Bar Harbor, Maine). For survival studies, mice were anesthetized and stereotactically injected with 1 × 10⁵ GBM cells (GBM30-FFL and U87ΔEGFR cells for nude mice and Gl261Nectin cells for C57BL/6 mice) into the right frontal lobe of the brain (2 mm lateral and 1 mm anterior to bregma at a depth of 3 mm). The GBM cells (GBM30FFL or Gl261Nectin) cells were allowed to grow for 5 days, and animals were subsequently randomly divided into groups that were injected intratumorally either with 2 × 10⁵ PFU oHSV (OV-CDH1 or OV-Q1) in 3 µl of HBSS or with vehicle. Mice were subsequently monitored frequently for GBM disease progression and were sacrificed when they became moribund with neurologic impairments and obvious weight loss. For the mechanism study, nude mice were stereotactically injected with 1 × 10⁵ GBM30-FFL cells into the same site of brain mentioned above. The GBM cells were allowed to grow for 10 days and the mice were subsequently intratumorally injected with 2 × 10⁵ PFU oHSV (OV-CDH1 or OV-Q1) or vehicle. 3 days after oHSV injection the mice were sacrificed to harvest brain for the following flow cytometry assay and IHC assay. Survival studies involving depletions of immune cells were performed using the same Gl261N4 GBM model. As GBM is a heterogeneous cancer, efficacy of OV-CDH1 was determined in additional models. Although the U87ΔEGFR GBM model is more aggressive due to the constitutively activated EGFR pathway, significant enhancement of OV therapy by OV-CDH1 vs. OV-Q1. GL261 tumors was observed that closely mimiced GBM phenotypes. Tumors established from GL261 cells recapitulated many characteristics of GBM. Histologic analysis revealed pleomorphism, pseudopalisading necrosis, and angiogenesis (Oh et al., 2014, Journal of translational medicine 12, 107). The GL261 murine model perhaps has been the most extensively used for preclinical testing of immunotherapeutic approaches for GBM (Maes et al., 2001, Cancer immunology, immunotherapy : CII 60, 153-160). NK cells were depleted by i.v. injections of NK1.1 neutralizing antibody twice, once on the day before virus injection and once two days after the virus injection. Macrophages were depleted by i.v. injections of clodronate liposomes using the same schedule as the NK cell depletion studies. CD4 and CD8 T cells were depleted by three-time i.v. injections of anti-CD4 and anti-CD8 neutralizing antibodies (Bio X Cell), respectively, once every three days beginning the day before virus injection. Corresponding control antibodies and liposomes were used as controls.

### Western blot and quantitative PCR

Western blot was performed as previously described with the primary antibody against hE-cadherin (DECMA-1), hN-cadherin (ab18203), beta-actin (sc-1616), and the corresponding secondary antibodies (*See* Deng et al., 2015, Immunity 42(3), 457-470; He et al., 2013, Blood121(23), 4663-71).

### Immunohistochemistry assay

Mouse brains isolated from OV-CDH1-, OV-Q1- or vehicle-treated GBM nude mice were placed in 10% buffered formalin for a minimum of 72h. After paraffin embedding, 4 µm-thick sections were cut from the blocks. H&E staining and IHC staining with anti-HSV antibodies were performed. The stained slides were mounted and viewed with a Zeiss microscope (AXIO observer.Z1).

Immunofluorescence studies were performed with OV-Q1- and OV-CDH1-infected human (U251) and mouse (Gl261N4) GBM cells at 48 hpi. Antibodies recognizing E-cadherin (clone HECD-1) and N-cadherin (clone D4R1H, recognizing both human and mouse N-cadherin) were used to stain the cells and determine the location of human E-cadherin, human N-cadherin and mouse N-cadherin.

### IFN-γ release assay

Human primary NK cells were incubated with target cells at the ratio of 1:1 in 96-well V bottom plates for 18 h. Cell-free supernatants were harvested and assayed for IFN- γ secretion by enzyme-linked immunosorbent assay (ELISA) using a kit from R&D Systems (Minneapolis, MN) in accordance with the manufacturer's protocol. Data depicted in the figures represent mean values of triplicate wells from one of three representative experiments with similar results.

### Statistics

For continuous endpoints that are normally distributed or normally distributed after data transformation (e.g. NK cell cytotoxicity, ⁵¹Cr release, IFN-γ level, viral production, etc), Student's *t* test was utilized to compare two independent conditions, and one-way ANOVA model was utilized to compare three or more conditions. For data with repeated measures from the same donor (e.g. CD107a expression in Figure 2E), linear mixed model was utilized to account for the variance-covariance structure due to repeated measures. For survival data, survival functions were estimated by the Kaplan-Meier method and compared by the log-rank test. All tests were two-sided. *P* values were adjusted for multiple comparisons by Holm's procedure. A *P* value of 0.05 or less was considered statistically significant.

### Example 2. OV-CDH1 infection introduces E-cadherin overexpression to GBM cells

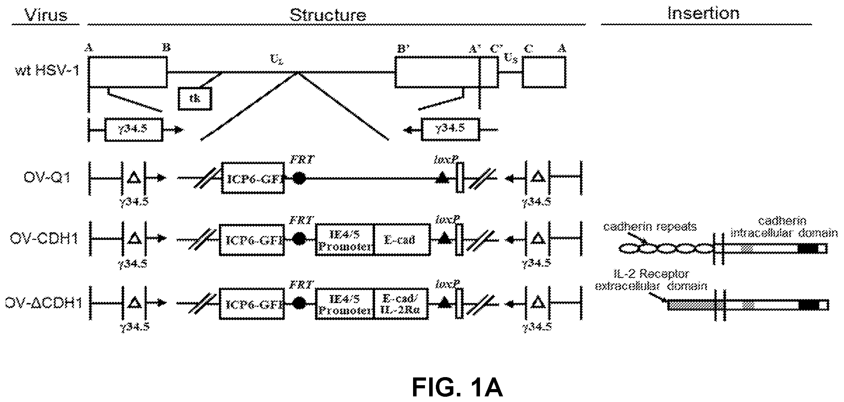

To introduce rapid overexpression of E-cadherin into oHSV-infected cells, a novel oHSV designated as OV-CDH1 was generated, carrying a human E-cadherin coding gene (CDH1) driven by the viral immediate early gene promoter, pIE4/5 from HSV-1. These are contained within the backbone of a double attenuated oHSV (OV-Q1), which has deficiency of the viral ribonucleotide reductase gene, ICP6, *e.g.,* inactivated by insertion of an EGFP gene, and deletions of two copies of the viral neurovirulence gene (ICP34.5) (Fig. 1A) (Terada et al., 2006, Gene Therapy 13, 705-714). These two genetic disruptions respectively resulted in a selective viral replication in tumor cells, as proliferating tumor cells instead of normal cells compensate for the loss of ICP6 by the host ribonucleotide reductase, which is not present in normal cells, and a reduction in neurovirulence (Mineta et al., 1995, Nature Medicine 1, 938-943). The FDA-approved Imlygic for the treatment of melanoma shares a similar backbone and expresses GM-CSF (Pol et al., DATE, Oncoimmunology 5, e1115641). Genetic maps of wild-type human HSV-1, parental oHSV (OV-Q1), and the recombinant OV-CDH1 are shown in Figure 1A. A mutant version of OV-CDH1, designated as OV-ΔCDH1 was previously generated. OV-ΔCDH1 expressed a fusion protein (E-cad/IL-2Rα), in which the extracellular domain of E-cadherin was replaced with that of the interleukin-2 receptor alpha chain (IL-2Rα) while the cytoplasmic domain of E-cadherin remained intact (Gottardi et al., 2001, J. Cell Biol. 153, 1049-1060 (2001). The genetic map of OV-ΔCDH1 is shown in FIG 1A.

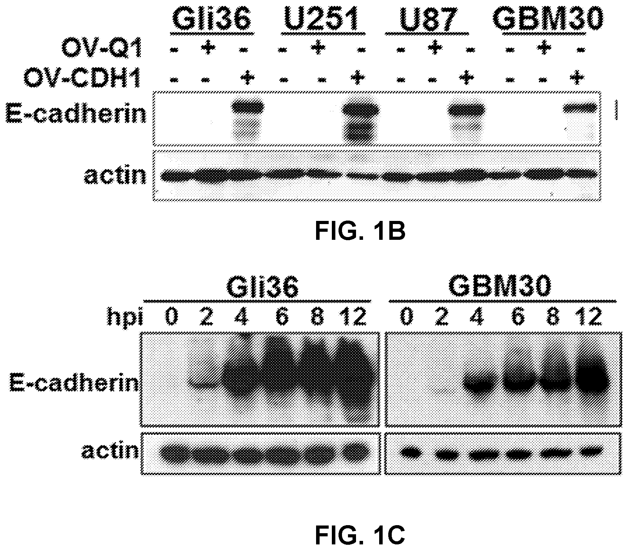

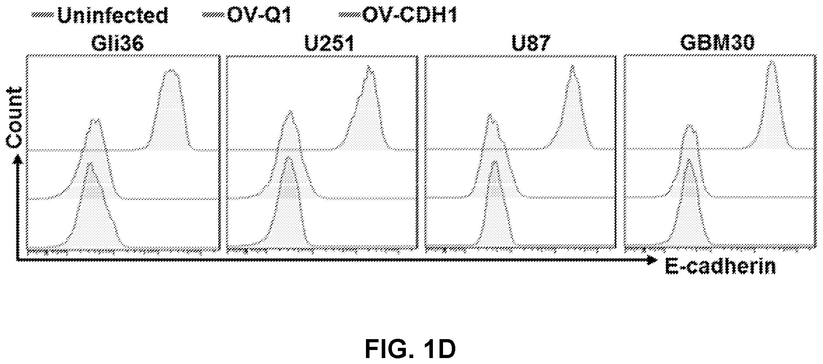

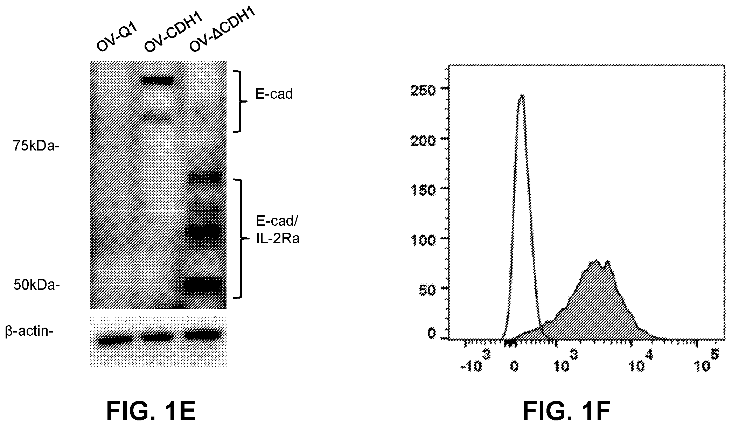

Validation of E-cadherin expression was performed in GBM cell lines, Gli36, U251, and U87 as well as patient-derived GBM30 spheroid cells. These GBM cells were infected with OV-Q1 or OV-CDH1 at a multiplicity of infection (MOI) of 5. E-cadherin overexpression in Gli36, U251, and U87 and patient-derived GBM30 spheroid cells was measured by western blotting at 8 hpi (Fig. 1B). The results showed that endogenous E-cadherin expression was absent prior to infection or upon infection with OV-Q1. In contrast, after being infected with OVCDH1, all the tested cells strongly expressed ectopic E-cadherin. And this ectopic E-cadherin could be detected as early as 2 hpi, likely due to the viral immediate early promoter, pIE4/5 used to express the E-cadherin gene by this virus (Fig. 1C). Flow cytometry analysis confirmed the western-blot findings and also demonstrated the cell surface location of the ectopic E-cadherin (Fig. 1D). To detect expression of mutant E-cadherin (E-cad/IL-2Rα) from OV-ΔCDH1, cell lysates of OV-CDH1- and OV-ΔCDH1-infected U251 cells harvested at 8 hpi were immunoblotted with an antibody against the cytoplasmic domain of E-cadherin. The protein sizes of E-cad/IL-2Rα were smaller than those of wild-type E-cadherin (Fig. 1E). These data were validated by a flow cytometric analysis with an anti-CD25 (IL-2Rα) antibody (Fig. 1F), which detected cell surface expression of E-cad/IL-2Rα.

### Example 3. E-cadherin protects OV-CDH1-infected GBM cells from being killed by NK cells

The sensitivity of OV-CDH1-infected cells to NK cell cytotoxicity using Chromium-51 (⁵¹Cr) release assays was tested. As shown in Figure 2A, both OV-Q1 and OV-CDH1 infections increased the susceptibility of GBM cells, Gli36, U251, U87, and GBM30, to NK cell cytotoxicity. However, OV-CDH1-infected cells were killed moderately but significantly less compared to OV-Q1-infected cells when bulk human primary NK cells with about 50% KLRG1⁺ cells were used (Fig. 2A, Fig. 17). The inhibitory effect was completely abolished when OV-ΔCDH1-infected Gli36 cells were used as target cells. (Fig. 1G). Next, human primary NK cells were separated into two populations by FACS-sorting and repeated the cytotoxicity experiment. When KLRG1⁻ NK cells were used as effector cells, there was no difference in killing of OV-Q1- and OV-CDH1-infected GBM cells (Fig. 2B). However, for same-paired donors, the activation reduction effect of OV-CDH1-infected cells on KLRG1⁺ NK cells was substantially greater than that of bulk NK cells (Fig. 2A). Approximately half of the NK cell killing induced by oHSV infection was suppressed when OV-CDH1 were compared with OV-Q1 (Fig. 2B). These findings were validated by flow cytometric analyses on bulk NK cells consisting of both KLRG⁺ and KLRG1⁻ cells, when gating on KLRG1⁺ and KLRG1⁻ cells to assess the expression of CD107a, a marker for degranulation that was previously shown to correlate with NK cell cytotoxicity (Fig. 2C & 2D) (Alter et al., 2004, J. Immunol. Meth. 294, 15-22). Degranulation of KLRG1⁺ cells was decreased upon co-culture with OV-CDH1-infected cells when compared with the co-culture with OV-Q1-infected cells; however, no difference in CD107a expression was observed between KLRG1⁻ NK cells co-cultured with OV-CDH1-infected GBM cells and with OV-Q1-infected GBM cells (Fig. 2C & 2D). Compared with OV-Q1 infected cells, co-culture with OVCDH1 infected cells induced less IFN-γ secretion of KLRG1⁺ NK cells. No difference in IFN-γ secretion was found with KLRG1⁻ NK cells co-cultured with OV-CDH1 or OV-Q1 infected cells (Fig. 2E).

### Example 4. Cell-cell fusion facilitates viral spread of OV-CDH1

For testing viral spread performance of OV-CDH1, monolayers of Gli36 and U251 cells were infected with OV-Q1 or OV-CDH1 at an MOI of 0.005. Infected cells were then imaged by fluorescence microscopy at 24, 48 and 72 hpi to record the plaque forming process. As shown in Figure 3A, plaques formed by OV-CDH1 infection grew much faster than those formed by OV-Q1 infection both in U251 and Gli36 cells, as the plaques formed by OV-CDH1 infection were significantly larger in size than those formed by OV-Q1 infection (Fig. 3A). At 72 hpi, the maximum diameters of OV-CDH1 plaques were approximately 2.5 times longer than those of OV-Q1 plaques (Fig. 3B). These data provide evidence for the enhanced viral spread of OV-CDH1 over the parental oHSV, OV-Q1. Additionally, the plaque forming process was recorded with time-lapse, live-cell microscopy imaging. The plaque forming video showed that viral spread of OV-CDH1 was much faster than OV-Q1 in cultured U251 GBM cells.

A plaque morphologic change accompanied with enhanced viral spread of OV-CDH1 was observed. This morphologic change of OV-CDH1 plaque was more evident after distinctive staining for cell membrane and nucleus (Fig. 3C). OVQ1⁻ infected cells were mononuclear with a distinct membrane structure for each cell. However, in the central area of OV-CDH1 plaque the cell membrane structure disappeared with multiple nucleates inside, indicating that the OV-CDH1-infected cells have fused together as multinucleated cells (Fig. 3C). A time-lapse plaque-forming video was also taken after infected cells were labeled with celltracker^{™} to show morphologic change of OV-CDH1 plaques. The video showed that during the process of forming OV-CDH1 plaques, GFP-positive (OV-CDH1-infected) cells directly fused with GFP negative (uninfected cells) and turn the latter into GFP positive cells. In contrast, OV-Q1-infected cells could not fuse with uninfected or infected neighboring cells. Caspase-3/7 staining showed that this cell fusion process was initiated with live cells, not dead cells (Fig. 18). Since cell staining was required for these experiments, liquid media were used to culture cells. However, the actual GBM microenvironment is much more dense than liquid media and has limited space for free viral particles to spread. To better mimic the GBM microenvironment, semi-solid media (1.2% methylcellulose) were also used to repeat the plaque forming experiment. Results indicated that the size of OV-CDH1 plaques formed in semi-solid media were larger than those formed in liquid media (Fig. 19).

### Example 5. Cadherin interaction contributes to cell-to-cell infection of OV-CDH1

To validate that ectopically expressed E-cadherin in OV-CDH1-infected cells contribute to enhanced viral spread, the extracellular domain of E-cadherin was blocked by treating the infected cells with an E-cadherin neutralizing antibody that is able to disrupt E-cadherin adhesion complexes, followed by a plaque forming assay (Vestweber et al., 1985, The EMBO Journal 4, 3393-3398; Ozawa et al., 1990, Mechanisms of Development 33, 49-56 ;Qian et al., 2004, The EMBO Journal 23, 1739-1748). HSV-1 glycoprotein D (gD) neutralizing antibody was included as a positive control. As predicted, gD antibody treatment almost completely blocked plaque formation after OV-CDH1 or OV-Q1 infection (Fig. 4A). However, E-cadherin neutralizing antibody treatment resulted in a significant plaque size reduction of OV-CDH1 but not of OV-Q1 (Fig. 4A & 4B). To further validate the specific role of the extracellular domain of E-cadherin in this process, OV-ΔCDH1 was used to repeat the plaque forming experiment. The results showed that mutant E-cadherin (E-cad/IL-2Rα), which lacks the extracellular domain of E-cadherin, could not induce cell-cell fusion nor enhancement of viral spread (FIG. 24A). There was no significant difference observed between OV-ΔCDH1 plaques and OV-Q1 plaques (FIG. 24B). Collectively, these results indicated that the extracellular domain of E-cadherin plays a direct role in enhancing viral spread of OV-CDH1. These results are consistent with a conclusion that overexpression of E-cadherin can enhance E-cadherin interaction between neighboring OVCDH1-infected cells and then facilitate membrane fusion between the cells, leading to enhanced viral spread.

N-cadherin is expressed in GBM cells, although these cells do not express E-cadherin (Fig. 1B and Fig. 4C). Immunofluorescence microscopy experiments with the OV-Q1- and OV-CDH1-infected human GBM cells (U251) showed that E-cadherin was only expressed in OV-CDH1-infected cells (Figure 20) and located on the cell surface (FIG. 24C). Co-localization of human E-cadherin and human N-cadherin was observed in OV-CDH1-infected U251 cells (FIG. 24C). Co-localizations of human E-cadherin and human N-cadherin distributed along the interface between OV-CDH1-infected cells and uninfected cells (FIG. 24C), where the cell-cell fusion occurred. To determine whether interaction between E-cadherin and N-cadherin was sufficient to lead to the observed cell fusion between an OV-CDH1-infected cell and an uninfected cell, N-cadherin expression was knocked down in Gli36 cells with shRNA and a plaque forming assay was performed (Fig. 4C). The results showed that knockdown of N-cadherin resulted in a significant plaque size decrease of OV-CDH1, but there was no significant change in the plaque size of OV-Q1 (Fig. 4D & 4E). This result implied that the interaction between E-cadherin and N-cadherin facilitates cell-cell fusion between OV-CDH1- infected and uninfected GBM cells, leading to direct cell-to-cell infections and thus enhanced viral spread of OV-CDH1.

### Example 6. Increased viral production of OV-CDH1

To test viral production capacity of OV-CDH1, U251 and Gli36 cells were infected with OV-CDH1 or OV-Q1 at an unsaturated infection MOI, 0.005. Virus production at 24, 48, 72 and 96 hours post-infection (hpi) was measured by plaque assays. The results showed that after the unsaturated infection (MOI = 0.005), OV-CDH1 produced significantly more virus than OV-Q1 (Fig. 5A & 5B). At 48 hpi, virus production of OV-CDH1 with Gli36 and U251 cells were approximately 17-fold and 13-fold higher than OV-Q1, respectively (Fig. 5A & 5B). This result suggested that enhanced viral spread of OV-CDH1 dramatically increased OV-CDH1 production. Next, virus production of OV-CDH1 and OV-Q1 was compared with saturated infections (MOI = 5), *i.e.,* all the cells being successfully infected after a primary infection, for which virus spread would not be expected to play a role. A 1.5 to 2-fold higher viral production of OV-CDH1 was observed at 24 hpi, however no significant differences were observed on viral production of OV-CDH1 and OV-Q1 at the later time points (48, 72 and 96 hpi) (Fig. 5C & 5D). This result suggested that in addition to enhanced viral spread, enhancement of viral entry may also contribute to higher viral production of OV-CDH1 (Fig. 5C & 5D).

### Example 7. Accelerated viral entry of OV-CDH1

As mentioned above, accelerated viral entry can be a reason for increased viral production of OV-CDH1 after saturated infection. Similar to cell-cell fusion, viral entry is also a membrane fusion process. Different from cell-cell fusion, however, membrane fusion during viral entry occurs between the viral membrane and the cellular membrane. E-cadherin located on the cell membrane was shown to facilitate the cell-cell fusion after OV-CDH1 infection (Fig. 3C). Immunoblotting assays on lysates of purified OV-CDH1 and OV-Q1 viral particles confirmed that overexpressed E-cadherin was packaged in OV-CDH1 viral particles, but not in OV-Q1 viral particles (Fig. 6A). To determine the viral membrane location of E-cadherin, an immunoprecipitation (IP) assay was performed with purified viral particles. Upon incubation with anti-E-cadherin-coated agarose beads, only OV-CDH1 virus was pulled down with the beads. E-cadherin and gD were both detected in the OV-CDH1 but not OV-Q1 pull-down fraction by anti-E-cadherin-coated agarose beads (Fig. 6B). Agarose beads were added from the abovementioned IP experiment to the monolayer of Gli36 cells. Only beads incubated with OV-CDH1 contained infective viruses, evidenced by plaque formation (Fig. 6C). This result suggested that E-cadherin was loaded on the surface of the viral particle of OV-CDH1 but not OV-Q1. Viral entry speed of OV-CDH1 and OV-Q1 was then tested. For this purpose, Gli36 cells were infected with OV-CDH1 or OV-Q1 at the MOI 0.005. At 5, 10, 20, 40, 60, 90, 120, 150, 180, 210, 240, 300 and 360 mins after infection, the infective media were removed and the cells were washed gently twice with PBS before overlay media were replaced. At 48 hpi, the plaque numbers were counted and "viral entry-time" curves were established to evaluate viral entry speed. Results showed that the viral entry speed of OV-CDH1 was faster than that of OV-Q1 (Fig. 6D).

### Example 8. OV-CDH1 shows an improved efficacy on GBM virotherapy in vivo

In order to evaluate the efficacy of OV-CDH1 on GBM therapy *in vivo,* a xenograft GBM mouse model was established by intracranially injecting GBM patient-derived cells, GBM30,_or U87ΔEGFR into athymic nude mice (Han et al., 2015, Cancer Research 75, 5273-5282). GBM30 cells were modified to express a luciferase gene for *in vivo* imaging (Han et al., 2015, Cancer Res. 75, 5273-5282). Figure 7A describes the experimental timeline for this *in vivo* study. Five days after tumor implantation, the animals were randomly assigned and intratumorally injected with OV-CDH1, OV-Q1, or vehicle. At day 10, the xenograft GBM mice underwent luciferase-based imaging to evaluate the progress of GBM. The results showed that OV-CDH1 was much more effective than OV-Q1 on treating GBM *in vivo* (Fig. 7B & 7C). Although OV-Q1 treatment slowed the progress of GBM, approximately 50% of luciferase signals were detectable in the OV-Q1-treated mice compared to vehicle-treated controls. In contrast, all the imaged OV-CDH1-treated mice exhibited little to no detectable luciferase signal. Survival analysis indicated that the median survival time of vehicle control mice was 24 days. The OV-Q1 treatment prolonged the median survival time to 34 days. In contrast, only one mouse from the OV-CDH1 group died on day 43 and all other mice survived over than 90 days without GBM symptoms and were euthanized at the end of the experiment (Fig. 7D). These data were confirmed by H&E staining of the brain sections harvested 10 and 20 days after tumor implantation in a repeated experiment. Tumor sizes of OV-Q1 treated mice were smaller than those of control mice, but the tumors were still solid and compact (Fig. 7E). In OV-CDH1 treated mice, however, no solid tumor were observed at the injection site and other places (Fig. 7E). The U87ΔEGFR GBM model is more aggressive than the GBM30 model with a median survival time of 16 days without treatment. In this model, OV-CDH1-treated mice still survive significantly longer than OV-Q1-treated mice (Fig. 7G).

The immune-competent GBM mouse model was better for evaluating efficacy of oHSV on treating GBM. However, it is challenging to establish an immunocompetent GBM model to study oHSV, because human GMB cells cannot effectively develop tumor in immune competent mouse and, although mouse GBM cells can develop tumor, most murine GBM cells are resistant to oHSV infection. (Fig. 9A) This problem was resolved by expressing human Nectin-1, a human herpesvirus entry receptor, in the murine GBM cell line Gl261.

The susceptibility of the engineered cell line, named Gl261Nectin, to oHSV infection was increased after this modification, although still 5-10 times lower than that of human GBM cells (Fig. 9). Consistent with previous data showing that E-oHSV has enhanced viral entry to and viral spread in GBM cells, E-oHSV also showed enhanced viral infection in Gl261Nectin cells compared to control o-HSV (Figs. 10A & 10B). Human E-cadherin was found expressed in OV-CDH1-infected but not OV-Q1-infected Gl261N4 cells (Figure 21) and expression was on the cell surface (Fig. 10C). Co-localization of human E-cadherin and murine N-cadherin was observed along the interface between the OV-CDH1-infected cells and the uninfected cells (Fig. 10C). By intracranially injecting Gl261Nectin cells into immunocompetent wild-type C57BL/6 mice, a GBM immunocompetent mouse model was established. Treatment of these mice with OV-CDH1 significantly prolonged the survival of immunocompetent mice bearing Gl261Nectin cells, and the median survival time was increased from 25 days to 42.5 days compared to the OV-Q1 control virus (Fig. 7F). OV-ΔCDH1 was also used to perform a survival study with the Gl261N4 immunocompetent GMB model. The results showed that without the extracellular domain of E-cadherin, OV-ΔCDH1 did not show significant improvement in treating GBM when compared to OV-Q1 (Fg. 1H).

### Example 9. OV-CDH1 treatment improves immune cell infiltration, in vivo viral spread and oncolysis

In order to further characterize the effects of OV-CDH1 in the *in vivo* tumor environment, xenograft GBM animal studies were repeated with a slightly altered timeline from the previously described experiment. This experiment was to evaluate the immune cell infiltration, activation, intratumoral viral spread as well as oncolysis. GBM30 cells were implanted and then 10 days were allowed to pass, followed by a shorter oHSV treatment course of 3 days, avoiding complete tumor eradication especially by OV-CDH1 (Fig. 8A). 3 days after oHSV infection, mouse brains were harvested for flow cytometry and histology study. Flow cytometry results showed that 3 days after viral infection, OV-Q1 and OV-CDH1 both recruited NK cells (CD3⁻NKp46⁺ lymphocyte) and macrophages (D115⁺CD45^{high}CD11b⁺ monocyte) to the brains (Fig. 8B). Brains treated with OV-CDH1 featured significantly more NK cells than OV-CDH1 treatment, notable in Figure 8B. However, no significant difference was found with macrophage and microglia (CD115⁺CD45^{low}CD11b⁺) recruitment by OV-CDH1 or OV-Q1 (Fig. 8B). The flow cytometry assay of CD107a showed that OV-CDH1 specifically inhibited the cytotoxicity of KLRG1 positive but not negative NK cells *in vivo,* which is consistent with the *in vitro* finding (Fig. 8C & 8D and Fig. 2C & 2D). H&E training showed improved tumor rejection of OV-CDH1, consistent with data in Fig 7E (Fig. 8E). IHC staining with anti-HSV showed enhanced viral spread and viral distribution of OV-CDH1, accompanying with improved oncolysis (Fig. 8F). Viral production assays showed that OV-CDH1-treated brains had 7 times more viral DNA detected than those treated with OV-Q1 (FIG 8G). In the immunocompetent GBM model, compared to treatment with OV-Q1, treatment with OV-CDH1 resulted in recruitment of significantly more NK cells as well as macrophages 3 days after administration (Fig. 8H). Virus production of OV-CDH1 was also over 10 times higher than that of OV-Q1 (Fig. 8I), and treatment with OV-Q1 resulted in greater infiltration of NK cells and/or macrophages as mentioned above. When focused on adaptive immune cells, OV-Q1 and OV-CDH1 treatments were both found to have significantly increased intracranial infiltrations of CD4 and CD8 T cells on day 3 and day 7 after OV injection. However, no significant difference in T cell infiltration was observed between OV-Q1 and OV-CDH1 (Figs. 8J and 8K). These findings confirmed improved efficacy of OV-CDH1 on treating GBM.

### Example 10: In vivo enhancement of OV-CDH1 virotherapy by E-cadherin expression is partially attributed to NK cell inhibition

To study the respective contributions of different immune cells in the improved efficacy of OV-CDH1 over OV-Q1, the survival studies using the Gl261N4 immune competent GBM model were repeated with or without depletion of NK cells, macrophages, CD4⁺, or CD8⁺ T cells. Efficacies of OV-CDH1 vs. OV-Q1 were compared in the presence of NK cells, where both viral spread and NK inhibition presumably play a role in the enhanced efficacy of OV-CDH1, and in the absence of NK cells, where only viral spread may play a role. The results showed that the difference between OV-CDH1 and OV-Q1 under the NK cell depletion circumstance was smaller than when NK cells were present (Fig. 22A), indicating that NK cell inhibition at least in part plays a role in improving the efficacy of OV-CDH1. When NK cells were depleted, such that only enhancement of viral spread would play a role, the difference of efficacy between OV-CDH1 and OV-Q1 was still significant, suggesting that enhanced viral spread also plays a role in improving the efficacy of OV-CDH1. Macrophage depletion significantly improved the efficacy of OV-Q1 and resulted in the same trend in OV-CDH1 (Fig. 22B). However, macrophages did not significantly contribute to improved efficacy of OV-CDH1, as the difference between OV-CDH1 and OV-Q1 under macrophage depletion circumstances was similar to their difference when macrophages were present. This might be due to the fact that the KLRG1⁺ NK cell subset does not produce substantial IFN-y, a cytokine that activates macrophages. The potential role of CD8 and CD4 T cells were also investigated. The results showed that, unlike NK cells and macrophages, CD8 T cell depletion seemed to lower efficacy of both OV-Q1 and OV-CDH1 for the treatment of GBM (Fig. 22C). However, CD4 T cell depletion had no significant influence on the efficacies of OV-CDH1 and OV-Q1 (Fig. 22D).

### Safety profiling of OV-CDH1

OV-Q1 and OV-CDH1 both carry the HSV-1 TK gene, which makes the viruses sensitive to the antiviral acyclovir and its derivatives. A viral replication inhibition assay showed that acyclovir effectively inhibited the replication of OV-Q1 and OV-CDH1 with IC₅₀ of 1.67 (95% CI, 0.9992 to 6.913) and 2.67 (95% CI, 0.673 to 4.171) µM, respectively (Fig. 12A). For evaluating the *in vivo* safety of OV-CDH1, BALB/c mice were intravenously (i.v.) or i.c. injected with wild-type HSV-1 (F strain), OV-Q1, or OV-CDH1 at the dose of 1 × 10⁶ PFU, which is the maximum dose due to the limited administration volume for i.c. injection. The mice with i.v. injection of wild-type HSV-1 all died on the next day. The mice with i.c. injection of wild-type HSV-1 all died within 5 days of injection. Mice treated with OV-Q1 and OV-CDH1 through i.v. or i.c. injections all survived over 4 weeks until they were euthanized and not monitored further (Fig. 12B). To study the bio-distribution of OV-CDH1, BALB/c mice were i.v. injected with OV-Q1 or OV-CDH1 at the dose of 1 × 10⁶ PFU. IHC was performed with sections of the heart, liver, spleen, lung, kidney and brain of the mice treated with OV-Q1 or OV-CDH1 for 2 days using an antibody against HSV-1. The results showed that the bio-distribution of OV-Q1 and OV-CDH1 was similar (Table 1).

**Table 1. Virus distribution at 2 days after i.v. injection**

| Virus distribution | | | | | | |
|---|---|---|---|---|---|---|
| | Heart | Liver | Spleen | lung | Kidney | Brain |
| OV-Q1 | - | ++ | - | + | + | - |
| OV-CDH1 | - | ++ | - | + | + | - |

Viral particles of OV-Q1 and OV-CDHI were distributed *in vivo* primarily in the liver. Virus was also detected in lung and kidney tissue. However, no virus was detected in heart, brain, or spleen. No significant distribution difference was observed between OV-Q1 and OV-CDHI-treated mice (Table 1). To study a potential tropism change of OV-CDHI, plaque-forming assays were performed using six different human primary cells including oral fibroblasts (HorF), pulmonary microvascular endothelial cells (HPMEC), hepatic sinusoidal endothelial cells (HHSEC), pulmonary alveolar epithelial cells (HPAEpiC), neurons (HN), and neurons-midbrain (HN-mb). Vero and U251 cells were also included as control. The results showed that there was no substantial tropism difference between OV-Q I and OV-CDHI (Fig. 23).

Each embodiment herein described may be combined with any other embodiment or embodiments unless clearly indicated to the contrary. In particular, any feature or embodiment indicated as being preferred or advantageous may be combined with any other feature or features or embodiment or embodiments indicated as being preferred or advantageous, unless clearly indicated to the contrary.

### EQUENCE LISTING

| | |
|---|---|
| SEQ ID NO:1 | CDS of I cadherin 1 (CDH1) from *Homo sapiens* cadherin 1 (CDH1), transcript variant 1, mRNA, NCBI ReferenceSequence: NM 004360.4 (127..2775) |
| ATGGGCCCTTGGAGCCGCAGCCTCTCGGCGCTGCTGCTGCTGCAGGTCTCC TCTTGGCTCTGCCAGGAGCCGGAGCCCTGCCACCCTGGCTTTGACGCCGAGAGC TACACGTTCACGGTGCCCCGGCGCCACCTGGAGAGAGGCCGCGTCCTGGGCAG AGTGAATTTTGAAGATTGCACCGGTCGACAAAGGACAGCCTATTTTTCCCTCGAC ACCCGATTCAAAGTGGGCACAGATGGTGTGATTACAGTCAAAAGGCCTCTACGGT TTCATAACCCACAGATCCATTTCTTGGTCTACGCCTGGGACTCCACCTACAGAAA GTTTTCCACCAAAGTCACGCTGAATACAGTGGGGCACCACCACCGCCCCCCGCC CCATCAGGCCTCCGTTTCTGGAATCCAAGCAGAATTGCTCACATTTCCCAACTCCT CTCCTGGCCTCAGAAGACAGAAGAGAGACTGGGTTATTCCTCCCATCAGCTGCCC | |
| AGAAAATGAAAAAGGCCCATTTCCTAAAAACCTGGTTCAGATCAAATCCAACAAAG ACAAAGAAGGCAAGGTTTTCTACAGCATCACTGGCCAAGGAGCTGACACACCCCC TGTTGGTGTCTTTATTATTGAAAGAGAAACAGGATGGCTGAAGGTGACAGAGCCT CTGGATAGAGAACGCATTGCCACATACACTCTCTTCTCTCACGCTGTGTCATCCAA CGGGAATGCAGTTGAGGATCCAATGGAGATTTTGATCACGGTAACCGATCAGAAT GACAACAAGCCCGAATTCACCCAGGAGGTCTTTAAGGGGTCTGTCATGGAAGGT GCTCTTCCAGGAACCTCTGTGATGGAGGTCACAGCCACAGACGCGGACGATGAT GTGAACACCTACAATGCCGCCATCGCTTACACCATCCTCAGCCAAGATCCTGAGC TCCCTGACAAAAATATGTTCACCATTAACAGGAACACAGGAGTCATCAGTGTGGT CACCACTGGGCTGGACCGAGAGAGTTTCCCTACGTATACCCTGGTGGTTCAAGCT GCTGACCTTCAAGGTGAGGGGTTAAGCACAACAGCAACAGCTGTGATCACAGTCA CTGACACCAACGATAATCCTCCGATCTTCAATCCCACCACGTACAAGGGTCAGGT GCCTGAGAACGAGGCTAACGTCGTAATCACCACACTGAAAGTGACTGATGCTGAT GCCCCCAATACCCCAGCGTGGGAGGCTGTATACACCATATTGAATGATGATGGTG GACAATTTGTCGTCACCACAAATCCAGTGAACAACGATGGCATTTTGAAAACAGCA AAGGGCTTGGATTTTGAGGCCAAGCAGCAGTACATTCTACACGTAGCAGTGACGA ATGTGGTACCTTTTGAGGTCTCTCTCACCACCTCCACAGCCACCGTCACCGTGGA TGTGCTGGATGTGAATGAAGCCCCCATCTTTGTGCCTCCTGAAAAGAGAGTGGAA GTGTCCGAGGACTTTGGCGTGGGCCAGGAAATCACATCCTACACTGCCCAGGAG CCAGACACATTTATGGAACAGAAAATAACATATCGGATTTGGAGAGACACTGCCA ACTGGCTGGAGATTAATCCGGACACTGGTGCCATTTCCACTCGGGCTGAGCTGG ACAGGGAGGATTTTGAGCACGTGAAGAACAGCACGTACACAGCCCTAATCATAGC TACAGACAATGGTTCTCCAGTTGCTACTGGAACAGGGACACTTCTGCTGATCCTG TCTGATGTGAATGACAACGCCCCCATACCAGAACCTCGAACTATATTCTTCTGTGA GAGGAATCCAAAGCCTCAGGTCATAAACATCATTGATGCAGACCTTCCTCCCAAT ACATCTCCCTTCACAGCAGAACTAACACACGGGGCGAGTGCCAACTGGACCATTC AGTACAACGACCCAACCCAAGAATCTATCATTTTGAAGCCAAAGATGGCCTTAGA GGTGGGTGACTACAAAATCAATCTCAAGCTCATGGATAACCAGAATAAAGACCAA GTGACCACCTTAGAGGTCAGCGTGTGTGACTGTGAAGGGGCCGCTGGCGTCTGT AGGAAGGCACAGCCTGTCGAAGCAGGATTGCAAATTCCTGCCATTCTGGGGATTC TTGGAGGAATTCTTGCTTTGCTAATTCTGATTCTGCTGCTCTTGCTGTTTCTTCGG AGGAGAGCGGTGGTCAAAGAGCCCTTACTGCCCCCAGAGGATGACACCCGGGA CAACGTTTATTACTATGATGAAGAAGGAGGCGGAGAAGAGGACCAGGACTTTGAC TTGAGCCAGCTGCACAGGGGCCTGGACGCTCGGCCTGAAGTGACTCGTAACGAC GTTGCACCAACCCTCATGAGTGTCCCCCGGTATCTTCCCCGCCCTGCCAATCCCG ATGAAATTGGAAATTTTATTGATGAAAATCTGAAAGCGGCTGATACTGACCCCACA GCCCCGCCTTATGATTCTCTGCTCGTGTTTGACTATGAAGGAAGCGGTTCCGAAG CTGCTAGTCTGAGCTCCCTGAACTCCTCAGAGTCAGACAAAGACCAGGACTATGA CTACTTGAACGAATGGGGCAATCGCTTCAAGAAGCTGGCTGACATGTACGGAGG CGGCGAGGACGACTAG | |
| SEQ ID NO:2 | CDS of Human herpesvirus 1 UL39 (ICP6) from Human herpesvirus 1 strain F, complete genome, GenBank: GU734771.1 (86339..89752) |
| ATGGCCAGCCGCCCAGCCGCATCCTCTCCCGTCGAAGCGCGGGCCCCGGTTGG GGGACAGGAGGCCGGCGGCCCCAGCGCAGCCACCCAGGGGGAGGCCGCCGGG GCCCCTCTCGCCCACGGCCACCACGTGTACTGCCAGCGAGTCAATGGCGTGATG GTGCTTTCCGACAAGACGCCCGGGTCCGCGTCCTACCGCATCAGCGATAGCAAC TTTGTCCAATGTGGTTCCAACTGCACCATGATTATCGACGGAGACGTGGTGCGCG GGCGCCCCCAGGACCCGGGGGCCGCGGCATCCCCCGCTCCCTTCGTTGCGGTG ACAAACATCGGAGCCGGCAGCGACGGCGGGACCGCCGTCGTTGCATTCGGGGG AACCCCACGTCGCTCGGCGGGGACGTCTACCGGTACCCAGACGGCCGACGTCC CAGCCGAGGCCCTTGGGGGCCCCCCTCCTCCTCCCCGCTTCACCCTGGGTGGC GGCTGTTGCTCCTGTCGCGACACACGGCGCCGCTCTGCGGTATTCGGGGGGGA GGGGGATCCCGTCGGCCCCGCGGAGTTCGTCTCGGACGACCGGTCGTCCGATT CCGACTCGGATGACTCGGAGGACACCGACTCGGAGACGCTGTCACACGCCTCCT CGGACGTGTCCGGCGGGGCCACGTACGACGACGCCCTTGACTCCGATTCGTCAT CGGATGACTCCCTGCAGATAGATGGCCCCGTGTGTCGCCCGTGGAGCAATGACA CCGCGCCCCTGGATGTTTGCCCCGGGACCCCCGGCCCGGGCGCCGACGCCGGT GGTCCCTCAGCGGTAGACCCACACGCGCCGACGACAGGGGCCGGCGCTGGTCT | |
| TGCGGCCGATCCCGCCGTGGCCCGGGACGACGCGGAGGGGCTTTCGGACCCCC GGCCACGTCTGGGAACGGGCACGGCCTACCCCGTCCCCCTGGAACTCACGCCC GAGAACGCGGAGGCCGTGGCGCGCTTTCTGGGAGATGCCGTGAACCGCGAACC CGCGCTCATGCTGGAGTACTTTTGCCGGTGCGCCCGCGAGGAAACCAAGCGTGT CCCCCCCAGGACATTCTGCAGCCCCCCTCGCCTCACGGAGGACGACTTTGGGCT TCTCAACTACGCGCTCGTGGAGATGCAGCGCCTGTGTCTGGACGTTCCTCCGGT CCCGCCGAACGCATACATGCCCTATTATCTCAGGGAGTATGTGACGCGGCTGGT CAACGGGTTCAAGCCGCTGGTGAGCCGGTCCGTTCGCCTTTACCGCATCCTGGG GGTTCTGGTGCACCTGCGGATCCGGACCCGGGAGGCCTCCTTTGAGGAGTGGCT GCGATCCAAGGAAGTGGCCCTGGACTTTGGCCTGACGGAAAGGCTTCGCGAGCA CGAAGCCCAGCTGGTGATCCTGGCCCAGGCTCTGGACCATTACGACTGTCTGAT CCACAGCACACCGCACACGCTGGTCGAGCGGGGGCTGCAATCGGCCCTGAAGT ATGAGGAGTTTTACCTAAAGCGCTTTGGCGGGCACTACATGGAGTCCGTCTTCCA GATGTACACCCGCATCGCCGGCTTTTTGGCCTGCCGGGCCACGCGCGGCATGC GCCACATCGCCCTGGGGCGAGAGGGGTCGTGGTGGGAAATGTTCAAGTTCTTTT TCCACCGCCTCTACGACCACCAGATCGTACCGTCGACCCCCGCCATGCTGAACC TGGGGACCCGCAACTACTACACCTCCAGCTGCTACCTGGTAAACCCCCAGGCCA CCACAAACAAGGCGACCCTGCGGGCCATCACCAGCAACGTCAGTGCCATCCTCG CCCGCAACGGGGGCATCGGGCTATGCGTGCAGGCGTTTAACGACTCCGGCCCC GGGACCGCCAGCGTCATGCCCGCCCTCAAGGTCCTCGACTCGCTGGTGGCGGC GCACAACAAAGAGAGCGCGCGTCCGACCGGCGCGTGCGTGTACCTGGAGCCGT GGCACACCGACGTGCGGGCCGTGCTCCGGATGAAGGGGGTCCTCGCCGGCGAA GAGGCCCAGCGCTGCGACAATATCTTCAGCGCCCTCTGGATGCCAGACCTGTTTT TCAAGCGCCTGATTCGCCACCTGGACGGCGAGAAGAACGTCACATGGACCCTGT TCGACCGGGACACCAGCATGTCGCTCGCCGACTTTCACGGGGAGGAGTTCGAGA AGCTCTACCAGCACCTCGAGGTCATGGGGTTCGGCGAGCAGATACCCATCCAGG AGCTGGCCTATGGCATTGTGCGCAGTGCGGCCACGACCGGGAGCCCCTTCGTCA TGTTCAAAGACGCGGTGAACCGCCACTACATCTACGACACCCAGGGGGCGGCCA TCGCCGGCTCCAACCTCTGCACCGAGATCGTCCATCCGGCCTCCAAGCGATCCA GTGGGGTCTGCAATCTGGGAAGCGTGAATCTGGCCCGATGCGTCTCCAGGCAGA CGTTTGACTTTGGGCGGCTCCGCGACGCCGTGCAGGCGTGCGTGCTGATGGTGA ACATCATGATCGACAGCACGCTACAACCCACGCCCCAGTGCACCCGCGGCAACG ACAACCTGCGGTCCATGGGAATCGGCATGCAGGGCCTGCACACGGCCTGCCTGA AGCTGGGGCTGGATCTGGAGTCTGCCGAATTTCAGGACCTGAACAAACACATCG CCGAGGTGATGCTGCTGTCGGCGATGAAGACCAGCAACGCGCTGTGCGTTCGCG GGGCCCGTCCCTTCAACCACTTTAAGCGCAGCATGTATCGCGCCGGCCGCTTTC ACTGGGAGCGCTTTCCGGACGCCCGGCCGCGGTACGAGGGCGAGTGGGAGATG CTACGCCAGAGCATGATGAAACACGGCCTGCGCAACAGCCAGTTTGTCGCGCTG ATGCCCACCGCCGCCTCGGCGCAGATCTCGGACGTCAGCGAGGGCTTTGCCCC CCTGTTCACCAACCTGTTTAGCAAGGTGACCCGGGACGGCGAGACGCTGCGCCC CAACACGCTCCTGCTAAAGGAACTGGAACGCACGTTTAGCGGGAAGCGCCTCCT GGAGGTGATGGACAGTCTCGACGCCAAGCAGTGGTCCGTGGCGCAGGCGCTCC CGTGCCTGGAGCCCACCCACCCCCTCCGGCGATTCAAGACCGCGTTTGACTACG ACCAGAAGTTGCTGATCGACCTGTGTGCGGACCGCGCCCCCTACGTCGACCATA GCCAATCCATGACCCTGTATGTCACGGAGAAGGCGGACGGGACCCTCCCAGCCT CCACCCTGGTCCGCCTTCTGGTCCACGCATATAAGCGCGGACTAAAAACAGGGA TGTACTACTGCAAGGTTCGCAAGGCGACCAACAGCGGGGTCTTTGGCGGCGACG ACAACATTGTCTGCACGAGCTGCGCGCTGTGA | |
| SEQ ID NO:3 | CDS of Human herpesvirus 1 RL1 (ICP34.5) from Human herpesvirus 1 strain F, complete genome (487..1233) and complement (124965..125711) |
| ATGGCCCGCCGCCGCCATCGCGGCCCCCGCCGCCCCCGGCCGCC CGGGCCCACGGGCGCCGTCCCAACCGCACAGTCCCAGGTAACCTCCAC GCCCAACTCGGAACCCGCGGTCAGGAGCGCGCCCGCGGCCGCCCCGC CGCCGCCCCCCGCCAGTGGGCCCCCGCCTTCTTGTTCGCTGCTGCTGC GCCAGTGGCTCCACGTTCCCGAGTCCGCGTCCGACGACGACGATGACG ACGACTGGCCGGACAGCCCCCCGCCCGAGCCGGCGCCAGAGGCCCGG CCCACCGCCGCCGCCCCCCGCCCCCGGTCCCCACCGCCCGGCGCGGG | |
| CCCGGGGGCGGGGCTAACCCCTCCCACCCCCCCTCACGCCCCTTCCG CCTTCCGCCGCGCCTCGCCCTCCGCCTGCGCGTCACCGCAGAGCACCT GGCGCGCCTGCGCCTGCGACGCGCGGGCGGGGAGGGGGCGCCGGAG CCCCCCGCGACCCCCGCGACCCCCGCGACCCCCGCGACCCCCGCGAC CCCCGCGCGGGTGCGCTTCTCGCCCCACGTCCGGGTGCGCCACCTGGT GGTCTGGGCCTCGGCCGCCCGCCTGGCGCGCCGCGGCTCGTGGGCCC GCGAGCGGGCCGACCGGGCTCGGTTCCGGCGCCGGGTGGCGGAGGCC GAGGCGGTCATCGGGCCGTGCCTGGGGCCCGAGGCCCGTGCCCGGGC CCTGGCCCGCGGAGCCGGCCCGGCGAACTCGGTCTAA | |

## Claims

1. An oncolytic herpes simplex virus (HSV) comprising a heterologous gene encoding E-cadherin.

2. The oncolytic HSV of claim 1, wherein the heterologous gene is operably linked to an HSV promoter sequence that is selectively active during HSV replication, optionally wherein the HSV promoter is an HSV immediate early promoter.

3. The oncolytic HSV of claim 1 or 2, further comprising an ICP 6-inactivating mutation or deletion.

4. The oncolytic HSV of any one of claims 1 to 3, further comprising a γ 34.5 inactivating mutation or deletion.

5. The oncolytic HSV of any of claims 1 to 4, for use in a method of treatment.

6. The oncolytic herpes simplex virus (HSV) of any one of claims 1 to 5 for use in the treatment of a tumor.

7. The oncolytic HSV for use of claim 6, wherein the tumor is a solid tumor, optionally wherein the solid tumor is a glioblastoma, an ovarian tumor or a breast tumor.

8. The oncolytic HSV for use of claim 6 or 7, wherein the subject is a mammal or a human.

9. A pharmaceutical composition comprising the oncolytic HSV of any one of claims 1 to 4 and a pharmaceutically acceptable carrier or diluent.

10. The oncolytic HSV of any one of claims 1 to 4, the oncolytic HSV for use of any one of claims 5 to 8, or the pharmaceutical composition of claim 9, wherein the heterologous gene encoding E-cadherin comprises the polynucleotide as set forth in SEQ ID NO: 1.

## Patentansprüche

1. Onkolytisches Herpes-simplex-Virus (HSV), umfassend ein heterologes Gen, das E-Cadherin codiert.

2. Onkolytisches HSV nach Anspruch 1, wobei das heterologe Gen operativ mit einer HSV-Promotorsequenz verknüpft ist, die während der HSV-Replikation selektiv aktiv ist, optional wobei der HSV-Promotor ein HSV-Unmittelbar-Früh-Promotor ist.

3. Onkolytisches HSV nach Anspruch 1 oder 2, ferner umfassend eine ICP-6-inaktivierende Mutation oder Deletion.

4. Onkolytisches HSV nach einem der Ansprüche 1 bis 3, ferner umfassend eine γ 34.5-inaktivierende Mutation oder Deletion.

5. Onkolytisches HSV nach einem der Ansprüche 1 bis 4 zur Verwendung in einem Behandlungsverfahren.

6. Onkolytisches Herpes-simplex-Virus (HSV) nach einem der Ansprüche 1 bis 5 zur Verwendung bei der Behandlung eines Tumors.

7. Onkolytisches HSV zur Verwendung nach Anspruch 6, wobei der Tumor ein solider Tumor ist, optional wobei der solide Tumor ein Glioblastom, ein Ovarialtumor oder ein Brusttumor ist.

8. Onkolytisches HSV zur Verwendung nach Anspruch 6 oder 7, wobei das Subjekt ein Säugetier oder ein Mensch ist.

9. Pharmazeutische Zusammensetzung, umfassend das onkolytische HSV nach einem der Ansprüche 1 bis 4 und einen pharmazeutisch annehmbaren Träger oder Verdünnungsmittel.

10. Onkolytisches HSV nach einem der Ansprüche 1 bis 4, onkolytisches HSV zur Verwendung nach einem der Ansprüche 5 bis 8 oder pharmazeutische Zusammensetzung nach Anspruch 9, wobei das heterologe Gen, das E-Cadherin codiert, das in SEQ ID NO: 1 angegebene Polynukleotid umfasst.

## Revendications

1. Virus oncolytique de l'herpès simplex (HSV) comprenant un gène hétérologue codant pour la E-cadhérine.

2. HSV oncolytique selon la revendication 1, dans lequel le gène hétérologue est lié de manière opérationnelle à une séquence promotrice du HSV qui est sélectivement active pendant la réplication du HSV, éventuellement dans lequel le promoteur du HSV est un promoteur précoce immédiat du HSV.

3. HSV oncolytique selon la revendication 1 ou 2, comprenant en outre une mutation ou une délétion inactivant ICP 6.

4. HSV oncolytique selon l'une quelconque des revendications 1 à 3, comprenant en outre une mutation ou une délétion inactivant γ 34.5.

5. HSV oncolytique selon l'une quelconque des revendications 1 à 4, pour une utilisation dans un procédé de traitement.

6. Virus oncolytique de l'herpès simplex (HSV) selon l'une quelconque des revendications 1 à 5 pour une utilisation dans le traitement d'une tumeur.

7. HSV oncolytique pour une utilisation selon la revendication 6, dans lequel la tumeur est une tumeur solide, éventuellement dans lequel la tumeur solide est un glioblastome, une tumeur ovarienne ou une tumeur mammaire.

8. HSV oncolytique pour une utilisation selon la revendication 6 ou 7, dans lequel le sujet est un mammifère ou un humain.

9. Composition pharmaceutique comprenant le HSV oncolytique selon l'une quelconque des revendications 1 à 4 et un support ou un diluant pharmaceutiquement acceptable.

10. HSV oncolytique selon l'une quelconque des revendications 1 à 4, le HSV oncolytique pour une utilisation selon l'une quelconque des revendications 5 à 8, ou la composition pharmaceutique selon la revendication 9, dans laquelle le gène hétérologue codant pour la E-cadhérine comprend le polynucléotide tel qu'indiqué dans SEQ ID NO : 1.
